(19) Europäisches Patentamt
European Patent Office
Office européen des brevets

(11) EP 3 786 642 A1

# (12) EUROPEAN PATENT APPLICATION

(43) Date of publication:
**03.03.2021 Bulletin 2021/09**

(51) Int Cl.:
***G01N 33/68*** *(2006.01)* ***G01R 33/60*** *(2006.01)*

(21) Application number: **19194752.2**

(22) Date of filing: **30.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ESPIRE Technologies GmbH**
**12487 Berlin (DE)**

(72) Inventors:
- **SCHNURR, Kerstin**
**16356 Ahrensfelde (DE)**
- **WATERSTRADT, Katja**
**12487 Berlin (DE)**
- **SCHOON, Boris**
**10437 Berlin (DE)**

(74) Representative: **Schulz Junghans Patentanwälte PartGmbB**
**Großbeerenstraße 71**
**10963 Berlin (DE)**

## (54) METHOD FOR FUNCTIONAL CHARACTERISTICS OF PROTEINS FOR CLASSIFICATION OF PATIENTS TO BE ASSOCIATED TO A SPECIFIC DISEASE

(57) The invention describes a method for functional characteristics of proteins for classification of patients to be associated to a specific disease performed in a computing device by applying biophysical parameters as an input to a marker linear combination, wherein the marker linear combination comprises a predefined combination of at least two biophysical parameters, using the marker linear combination to determine the input parameters relating to one or more of said predetermined diseases, and outputting a result according to the marker linear combination, wherein the marker linear combination comprises at least one of the biophysical parameters: a binding constant of a spin probe, a polarity surrounding a spin probe, an order parameter of a spin probe, a rotation correlation time of a spin probe, a spectral component from free spin probe molecules, a spectral component from spin probe on lipid-fraction of serum, or a geometry factor.

EP 3 786 642 A1

## Description

The present invention is related to a method for the classification of a sample. In particular, by means of the method a sample can be classified to be associated to a predetermined disease.

Embodiments generally relate to methods analysing extracellular fluids that contain a carrier protein, for example serum albumin and, more particularly, may relate to methods and associated devices for analysing the carrier protein to detect indicators of different liver diseases. In embodiments such analysis is carried out using electron spin resonance spectroscopy (ESR). Electron spin resonance (ESR) is also referred to as electron paramagnetic resonance (EPR).

The liver has a multitude of functions including blood detoxification, regulation of glucose metabolism, protein production, and production of hormones and bile. Amongst others, the liver produces albumin. Albumin is a carrier protein involved in the transport of fatty acids. Albumin is the main component in the transport system of blood, enabling the transport of fatty acids, tryptophan, bilirubin, calcium, steroid hormones, and other functionally significant active substances to the target cell, and being involved in binding and distribution of a variety of toxins (including those of endogenous origin). Albumin is a polypeptide (molecular weight 66.500 Da) comprised of 585 amino acids and having a high binding capacity for exogenous (drugs) and endogenous substances. Fatty acids are the primary physiological ligand of albumin. A capability of albumin to bind and transport drugs has been reported. Albumin can have an anti-oxidant activity.

Different liver diseases (also referred to as hepatic diseases) are well-known. A specific hepatic disease requires a specific related treatment. Therefore, not only an early detection of a hepatic disease is of importance for an effective treatment but also the differentiation between particular hepatic diseases such as e.g. acute on chronic liver failure (ACLF), cirrhosis without acute decompensation (CC) or cirrhosis with acute decompensation (AD). Under specific circumstances an AD evolves into an ACLF.

In the clinical practice, the abundance (or concentration) of particular indicators and/or values of so-called diagnostic scores are used for the diagnosis of hepatic diseases. Indicators and/or diagnostic scores can also provide prognostic information regarding a risk of deterioration of the disease. In the context of this application, a score is an indicator or a diagnostic score.

Indicators can be for instance the bilirubin concentration, the concentration of the alanine transaminase (ALT), the concentration of the aspartate transaminase (AST) or the concentration of albumin.

Examples of diagnostic scores are the CLIF-C-AD (Chronic Liver Failure Consortium Acute Decompensation) score, the Child-Pugh score (Child-Turcotte-Pugh score), the MELD (Model of End Stage Liver Disease) score, and the MELD-Na score. Scores can comprise at least one indicator.

The MELD score comprises information regarding the creatinine abundance, the bilirubin abundance, and the international normalised ratio (INR), which is an indicator for blood clotting. The MELD score is particularly used to estimate the mortality of patients in the terminal stage of liver cirrhosis.

The MELD-Na score further comprises information regarding the abundance of sodium (Na). Like the MELD score, the MELD Na score is particularly used to estimate the mortality of patients in the terminal stage of liver cirrhosis.

The Child-Pugh score comprises information regarding the bilirubin abundance, the albumin abundance (in particular the abundance of serum albumin), and the INR. Additionally, information regarding the presence of an ascites and the presence of an encephalopathy is included in the Child-Pugh score. This score is particularly used to estimate the stage of cirrhosis of a patient and/or estimate the course of the disease.

The CLIF-C-AD score comprises information regarding the age of the patient, the number of white blood cells (also referred to as leukocytes), the abundance of creatinine, the INR and the abundance of sodium (Na). The CLIF-C-AD score is particularly used to estimate the development of AD to ACLF and/or to give an estimate about the mortality of the AD patient.

A study has shown that functional characteristics of albumin can be used to classify a sample to be associated to a specific hepatic disease (Jalan, R. et al. (2009), "Alterations in the Functional Capacity of Albumin in Patients with Decompensated Cirrhosis is Associated with Increased Mortality", Hepatology, 50, 555-564). In particular, a binding coefficient of albumin and its detoxification efficiency can provide information to distinguish between a sample associated to a person without hepatic disease, a sample associated to a person with an ACLF and/or a sample associated to a person with a cirrhosis with no organ dysfunction.

By means of the indicators and/or diagnostic scores, a sample can be classified to be associated to a particular disease. The quality of the particular method, i.e. whether a score classifies a sample correctly, determines the performance of the method. In other words this means that the performance is a measure of the quality of the method. The performance depends on the sensitivity of the method as well as on the specificity of the method, in particular the quality of the method to classify healthy people to be healthy and sick people to be sick.

The sensitivity, also referred to as true positive rate, gives the number how many samples associated to a particular disease are classified to be associated to that particular disease. For example, this means that the sensitivity gives the portion of sick people whose disease has been identified correctly. The more samples (people) are classified correctly, the higher the sensitivity. The sensitivity depends in particular on the number of true positive.

The specificity, also referred to as true negative rate, determines the portion of samples being correctly classified to not show the particular disease (i.e. to be associated to a healthy person). This means for instance that a healthy person is classified to be healthy (i.e. not showing the particular disease). The specificity depends on the number of true negative. A further measure is the false positive rate (FPR), which is calculated as 1-specificity (or 100%-specificity). The FPR depends on the number false positive. It determines the portion of negative samples that are classified to be positive. For instance, it gives the portion of healthy persons that are classified to be sick.

Depending on the particular medical issue, it might be important to have a high sensitivity such that (almost) all sick people are detected by the method. Under different circumstances, it might be more important to have a high specificity while the sensitivity is less important.

A measure that takes both the sensitivity and the specificity into account is the receiver operating characteristics (ROC) curve, in particular the area under the ROC curve (AUROC). In general, it holds that the closer the AUROC is to one, the better the performance.

A method is of interest that can classify a sample, in particular a blood sample, to be associated to a specific hepatic disease, a stage of a hepatic disease and/or a predefined hepatic function in an easy and reliable manner. A method is of need that can provide a probability regarding the course of a disease, in particular a prognostic information regarding a risk of deterioration of a disease, in particular prognostic information regarding a risk of deterioration of a hepatic disease, a stage of a hepatic disease and/or a predefined hepatic function, based on the current sample. This comprises the possible passage from one diseases stage to another, i.e. the transition from AD to ACLF. It also comprises to give a probability whether a specific therapy would be successful or not. In particular, a method is of need with a good performance.

This is provided by the method according to claim 1. Embodiments of the present invention are stated in the corresponding sub claims and are described below.

A first aspect method performed in a computing device, the method comprising the steps of:

receiving at the computing device a plurality of spectra acquired from a corresponding plurality of aliquots containing a biophysiological carrier protein, wherein at least one of a concentration of a spin probe and a concentration of a polar reagent varies between the aliquots;

determining by the computing device biophysical parameters based on the received spectra;

applying by the computing device the biophysical parameters as an input to a marker linear combination, wherein the marker linear combination comprises a predefined combination of at least two biophysical parameters,

the computing device using the marker linear combination to determine the input parameters relating to one or more of said predetermined diseases, and

outputting a result according to the marker linear combination.

The marker linear combination comprises at least one of the biophysical parameters:

- a binding constant of a spin probe (KB),
- a polarity surrounding a spin probe (H),
- an order parameter of a spin probe (S),
- a rotation correlation time of a spin probe (T),
- a spectral component from free spin probe molecules (C3),
- a spectral component from spin probe on lipid-fraction of serum (C5), or
- a geometry factor (alpha).

The plurality of spectra can be a plurality of ESR spectra. An ESR spectrometer can be used to measure an ESR spectrum, in particular the plurality of ESR spectra.

In an embodiment, the biophysiological carrier protein is albumin, in particular serum albumin. In the context of the present application, for the sake of convenience, serum albumin is also referred to as albumin.

Albumin is produced in the liver. In case of a hepatic disease, the albumin concentration is reduced. Further, albumin can have an abnormal functionality in case of a hepatic disease. For instance, the ability to bind particular ligands, such as hormones, fatty acids and/or drugs, can be altered.

The properties of albumin to bind ligands can be determined by means of an ESR spectrum, in particular by means of a plurality of ESR spectra. The particular ESR spectrum and/or characteristic biophysical parameters derived from the ESR spectrum can be used.

This means that by means of a plurality of ESR spectra, a binding property of albumin can be determined that can be an indication of a particular hepatic disease.

The plurality of aliquots can comprise three aliquots.

In an embodiment, the polar reagent is an alcohol, in particular a $C_1$-$C_6$ alcohol. A $C_1$-$C_6$ alcohol is an alcohol with a hydrocarbon chain length, comprising 1, 2, 3, 4, 5, or 6 carbon atoms. According to an embodiment, the polar reagent is ethanol. In another embodiment, the polar reagent is DMSO. The polar reagent can act as a solvent for the spin probe and acts to modify the polarity of the mixture.

In an embodiment, the concentration of ethanol in each aliquot of the plurality of aliquots is different.

For each aliquot of the plurality of aliquots, a respective ESR spectrum of the plurality of ESR spectra is measured.

In an embodiment, the spin probe can be an organic molecule that possesses an unpaired electron. The spin probe can have the ability to bind to another molecule. In an embodiment, the spin probe is 16-doxyl stearic acid. 16-doxyl stearic acid has the following structure:

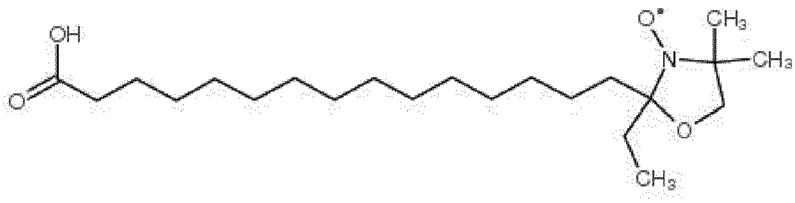

.

In an alternative embodiment, the spin probe is an alternative spin-labelled fatty acid. The spin probe can be 5-, 7-, 12- or 16-doxyl stearic acid or 16-doxyl stearate. According to an embodiment, the spin probe is a doxyl lauric acid, in particular 7-doxyl lauric acid. In the context of the application, spin probe is also referred to as spin label.

The concentration of the spin label can be different in the different aliquots.

In the context of the application, the solution with the lowest concentration of the polar reagent and/or the lowest concentration of the spin probe is referred to as solution A. The solution with the medium concentration of the polar reagent and/or the medium concentration of the spin label is referred to as solution B. The solution with the highest concentration of the polar reagent and/or the highest concentration of the spin label is referred to as solution C.

In the context of the present application, the first spin label (first spin probe) is also referred to as spin label 1 (spin probe 1), which reflects the spin probe molecules bound in high affinity binding sites. In the context of the present application, the second spin label (second spin probe) is also referred to as spin label 2 (spin probe 2) which reflects the spin probe molecules bound in low affinity binding sites.

Each biophysical parameter can be related to a particular biological property of albumin, e.g. a binding efficiency of albumin. A particular state of albumin can be characterised by the biophysical parameters. A combination of at least two biophysical parameters can advantageously characterise the actual state of albumin more accurately than a single biophysical parameter. Therefore, a marker linear combination comprising at least two biophysical parameters can be used to distinguish between normal (healthy) and abnormal (disease) states. It can also be used to classify samples to different disease states.

A biophysical parameter can be a spectral component. An ESR (EPR) spectrum consists of a plurality of sub-spectra. In the context of the application, a sub-spectrum is also referred to as spectral component. A spectral component can be a spectral component from spin probe bound to albumin with a high binding affinity, a spectral component from spin probe bound to albumin with a low binding affinity, a spectral component from free spin probe molecules, a spectral components from free spin probe in micelles, or a spectral component from spin probe on lipid-fraction of serum.

Further biophysical parameters can also be determined based on the measured spectra, in particular the measured EPR spectra. These include:

- The polarity (hydrophilicity) surrounding the spin label for the first/high affinity spectral component C1 and for the second/low affinity spectral component C2,
- Spin probe ordering,
- Spin probe effective correlation time,

- Intensity, or
- Geometry factor Alpha

The polarity (hydrophilicity) surrounding the spin label for the first/high affinity spectral component C1 and for the second/low affinity spectral component C2 may be referred to as H1 (for the C1 component) and H2 (for the C2 component) and are, in some sources, referred to as "P". P is defined as:

$$P = \frac{A - A_H}{A_W - A_H} \qquad A = \left(A_{\parallel} + 2A_{\perp}\right)/3$$

where $A_{\perp,\parallel}$ - are hyperfine splitting constant respectively perpendicular or parallel to the axis of external magnetic field as applicable C1 and C2 (the high and low affinity binding sites have different hyperfine splitting constants associated with them), $A_H$ is the hyperfine splitting constant for a spin probe in a hydrophobic medium, and $A_W$ is the hyperfine splitting constant for a spin probe in a hydrophilic medium (see Muravsky, V., Gurachevskaya, T., Berezenko, S., Schnurr, K., Gurachevsky, A. (2009): Fatty acid binding sites of human and bovine albumins: differences observed by spin probe ESR., Spectrochim Acta A Mol Biomol Spectrosc, 74, 42-47).

Spin probe ordering is associated with the angle of the spin label axis precession for the first and for the second component, S1 (for component C1) and S2 (for component C2), corresponding:

$$S = \frac{A_{\parallel} - A_{\perp}}{A_Z^0 - \left(A_X^0 + A_Y^0\right)/2} \cdot \frac{A_Z^0 + A_X^0 + A_Y^0}{A_{\parallel} + 2A_{\perp}}$$

Spin probe effective correlation time, $T_1$ (for component C1) and $T_2$ (for component C2). T can also be referred to as "T"):

$$\tau = 4.17 \cdot 10^{-10} \cdot \left(1 - \frac{A_{\parallel}}{A_{\parallel}^0}\right)^{-\frac{3}{2}} \cdot \frac{32}{A_{\parallel}^0}$$

The geometry factor alpha is calculated as the ratio of the amplitude of the left-most spectral component of the ESR spectrum and the magnitude of the last but one spectral component on the right hand side of the ESR spectrum. These two peaks are related by their g-factors. Alpha is the ratio of intensities at the position of the high-field line of unbound 16-doxyl-stearic spin label relative to the spectral intensity at the position of low-field line of the bound 16- DSA in the albumin binding sites with high affinity. Alpha depends on the raw data and is not dependent on particular assumptions and/or simplifications of the model for simulation of ESR spectra.

$T_{1Freed}$ is the correlation time for the first motional component calculated as follows:

$$\tau = 5.4 * 10^{-10} * \left(1 - \left(\frac{A_{max}}{A_{zz}}\right)\right)^{-1.36}$$

where $A_{zz}$=33,6G is the constant of hyperfine splitting projection for the first component onto Z-axis.

$T_{2Podolka}$ is the correlation time for the second motional component calculated as follows:

$$T_{2Podolka} = 5.95 * \Delta H_0 * \left(\sqrt{\frac{I_0}{I_{+1}}} + \sqrt{\frac{I_0}{I_{-1}}} - 2\right) * 10^{-10}$$

where $I_{-1}$, $I_0$ and $I_{+1}$ are corresponding peak-to-peak high-field, middle-field and low-field intensities of the C2 component of the ESR-spectrum.

$T_{GlobFreed}$ is the correlation time for the albumin globule, calculated as T1Freed/(S1)$^2$, wherein S1 is the ordering factor for the first motional component C1.

AppDissConst is the apparent constant of dissociation of the spin-label with albumin determined by:

$$K_0 = \frac{k_{-1}}{k_1} = \frac{[R][L]}{[RL]} = \frac{[R_0 - b]f}{b}$$

where R is the concentration of albumin, L is the concentration of the spin probe and RL is the concentration of the complex of both agents. $R_0$ is the estimated total binding capacity of albumin with the spin probe, determined by multiplying the concentration of albumin measured in the blood sample by 7 (the number of binding sites of fatty acids on albumin), f is the concentration of free (unbound) spin probe (determined (three times for A, B and C respectively) by multiplying C3 with concentration of the spin probe) and b is the concentration of bound spin probe (determined (again, three time for A, B and C respectively) by adding C1 and C2 and multiplying it with the concentration of the spin probe probe).

The binding constant KB is an apparent binding constant. It is the reciprocal of the apparent dissociation constant. The binding constant KB describes the global binding capability of albumin under the specific prevailing conditions. This means it describes the global binding capability of albumin for the actual concentration of the polar reagent. It can particularly describe the global binding capability of albumin for the actual albumin concentration. Therefore, KB of the present application differs from the binding constant published in the patent specification EP 1 269 213 B1 which was determined by a regression of ratios between relative amount of spin labelled molecules within the different binding sites to the concentration of the polar reagent. It also differs from the binding constants $K_{B1}$ and $K_{B2}$ discussed in the publication by Jalan and colleagues (Jalan, R. et al. (2009), "Alterations in the Functional Capacity of Albumin in Patients with Decompensated Cirrhosis is Associated with Increased Mortality", Hepatology, 50, 555-564) which described the binding coefficients of high-affinity sites and low affinity sites, respectively. Both were determined by a regression of ratios between relative amounts of spin labelled molecules within either high affinity or low affinity binding sites to the concentration of the polar reagent.

Correlation time $T_1$ of the albumin globule rotation and the correlation time $T_2$ of the spin probe motion relative to the globule (with the axis precession within the angle $\theta$) are calculated from the following equations:

$$\tau^{-1} = \tau_1^{-1} + \tau_2^{-1} \qquad \tau = \left(\frac{3cos^2\theta - 1}{2}\right)^2 \tau_1$$

Transport parameters of albumin (binding efficiency BE and detoxification efficiency DTE) are calculated from the following equation of binding constant for the three different concentrations of the ethanol:

$$C_{Unbound_DSA} = \frac{1}{K_B'} * \frac{C_{eth}}{C_{eth}^0 - C_{eth}} * \frac{C_{Bound_DSA}}{C_{Binding_sites} - C_{Bound_DSA}}$$

where $C_{eth}$ is the Ethanol concentration in the current sample, $C_{eth}^0$ is the Ethanol concentration of ethanol when 16-DSA completely dissociates from albumin and $K_B$' is the Albumin binding constant for LCFA (long chain fatty acids) at

$$C_{eth} = \frac{1}{2} * C_{eth}^0$$

The above discussed correlation times derivable from the ESR spectra provide information on the mobility of a spin probe attached at the respective binding sites and the affinity of protein for the spin probe. In addition, the dipolar interactions between spin probes bound to different parts of the protein can also be measured. Changes in the mobility and binding affinity of a spin probe, and the distribution of the spin probe on the albumin molecule, allows the functional and structural properties of a protein to be assessed. Comparison of the changes that occur to the mobility, binding

affinity, and distribution of a spin probe on albumin in normal healthy individuals with those changes observed in patients with a disease states can reveal unique alterations. This information can be of value in the diagnosis and monitoring of a disease. The biophysical parameters can provide information to classify samples to be associated to a particular liver disease.

In an embodiment, the rotation correlation time of a spin probe (T) and the order parameter of a spin probe (S) are related to each other. This is referred to as ration TG. The ratio TG1 is determined by $\tau_1/(S1)^2$, wherein S1 is the ordering factor for the first motional component C1 and TG2 is determined by $\tau_2/(S2)^2$, wherein S2 is the ordering factor for the second motional component C2.

A linear combination (lc) can have the form $\mathrm{lc} = \sum_{i=1}^{n} \mathrm{ki} * \mathrm{vi}$, wherein $k_i$ is a coefficient, and $v_i$ can be a biophysical parameter. According to an embodiment, n ≥ 2. This means that the linear combination can comprise at least two biophysical parameters. In an embodiment, the linear combination can comprise a combination of at least two biophysical parameters and a score. A score can be an indicator or a diagnostic score. An indicator can be the concentration or a quantity derived therefrom or relating thereto of a particular protein, such as bilirubin, the alanine transaminase (ALT), the aspartate transaminase (AST) or albumin. The diagnostic score can be the CLIF-C-AD score, the Child-Pugh score, the MELD score, and the MELD-Na score.

The marker linear combination comprises at least one of the biophysical parameters:

- a binding constant of a spin probe (KB),
- a polarity surrounding a spin probe (H),
- an order parameter of a spin probe (S),
- a rotation correlation time of a spin probe (T),
- a spectral component from free spin probe molecules (C3),
- a spectral component from spin probe on lipid-fraction of serum (C5), or
- a geometry factor (alpha).

According to an embodiment, the marker linear combination is a marker linear combination for classification of a sample to be associated to a predetermined disease.

According to an embodiment, the marker linear combination comprises the binding constant of a spin probe (KB). Alternatively, the marker linear combination can comprise the polarity surrounding a spin probe (H). In an embodiment, the marker linear combination comprises the order parameter of a spin probe (S). In another embodiment, the marker linear combination comprises the rotation correlation time of a spin probe (T). In a further embodiment, the marker linear combination comprises the spectral component from free spin probe molecules (C3). According to an embodiment, the marker linear combination comprises spectral component from spin probe on lipid-fraction of serum (C5). In an embodiment, the marker linear combination comprises the geometry factor (alpha).

According to an embodiment, the binding constant of a spin probe is a binding constant in solution A (KBA). The binding constant of a spin probe can be a binding constant in solution B (KBB). According to the invention, the binding constant of a spin probe can be the binding constant in solution C (KBC).

According to an embodiment, the spectral component from free spin probe molecules (C3) is the respective spectral component in solution A (C3A). The spectral component from free spin probe molecules can be a spectral component from free spin probe molecules in solution B (C3B). According to the invention, the spectral component from free spin probe molecules can be the spectral component from free spin probe molecules in solution C (C3C).

According to an embodiment, the spectral component from spin probe on lipid-fraction of serum (C5) is the respective spectral component in solution A (C5A). The spectral component from spin probe on lipid-fraction of serum can be a spectral component from spin probe on lipid-fraction of serum in solution B (C5B). According to the invention, the spectral component from spin probe on lipid-fraction of serum can be the spectral component from spin probe on lipid-fraction of serum in solution C (C5C).

The polarity surrounding of a spin probe can be the polarity surrounding of a spin probe 1 (H1). Alternatively, the polarity surrounding of a spin probe can be the polarity surrounding of a spin probe 2 (H2). The polarity surrounding of a spin probe can be the polarity surrounding of a spin probe 1 in solution A, in solution B or in solution C (H1A, H1B or H1C, respectively). The polarity surrounding of a spin probe can be the polarity surrounding of a spin probe 2 in

solution A, in solution B or in solution C (H2A, H2B or H2C, respectively).

The order parameter of a spin probe can be the order parameter of the spin probe 1 (S1). Alternatively, the order parameter of a spin probe can be the order parameter of spin probe 2 (S2). The order parameter of a spin probe can be the order parameter of the spin probe 1 in solution A, in solution B or in solution C (S1A, S1B or S1C, respectively). The order parameter of a spin probe can be the order parameter of a spin probe 2 in solution A, in solution B or in solution C (S2A, S2B or S2C, respectively).

The rotation correlation time of a spin probe can be the respective rotation correlation time of a spin probe 1 (T1). Alternatively, the rotation correlation time of a spin probe can be the rotation correlation time of probe 2 (T2). The rotation correlation time of a spin probe can be the rotation correlation time of spin probe 1 in solution A, in solution B or in solution C (T1A, T1B or T1C, respectively). The rotation correlation time of a spin probe can be the respective rotation correlation time of a spin probe 2 in solution A, in solution B or in solution C (T2A, T2B or T2C, respectively).

According to an embodiment, the ratio TG is considered. The ratio can be the respective ratio according to spin probe 1 (TG1). Alternatively, the ratio can be the respective ratio according to spin probe 2 (TG2). The ratio can be the ratio according to spin probe 1 in solution A, in solution B or in solution C (TG1A, TG1B or TG1C, respectively). The ratio can be the respective ratio with re a spin probe 2 in solution A, in solution B or in solution C (TG2A, TG2B or TG2C, respectively).

Each one of these biophysical parameters can contribute to the classification of a sample to be associated to a predetermined disease.

In an alternative embodiment, that the marker linear combination comprises at least one of the biophysical parameters:

- a binding constant of a spin probe (KB),
- a polarity surrounding a spin probe (H),
- an order parameter of a spin probe (S),
- a rotation correlation time of a spin probe (T),
- a spectral component from free spin probe molecules (C3), or
- a spectral component from spin probe on lipid-fraction of serum (C5).

According to an embodiment, the marker linear combination comprises at least one of:

- a spectral component from free spin probe molecules (C3),
- a spectral component from spin probe on lipid-fraction of serum (C5),
- a rotation correlation time of a spin probe (T),
- an order parameter of a spin probe (S), or
- a polarity surrounding a spin probe (H).

According to an embodiment, the marker linear combination comprises:

- a spectral component from free spin probe molecules (C3),
- a spectral component from spin probe on lipid-fraction of serum (C5), or
- a polarity surrounding a spin probe (H).

In an embodiment, the marker linear combination comprises at least one of:

- a combination of a spectral component from spin probe on lipid-fraction of serum (C5) and a spectral component from free spin probe molecules (C3),

- a combination of a spectral component from spin probe on lipid-fraction of serum (C5) and a geometry factor (alpha),
- a combination of a spectral component from free spin probe molecules (C3) and a geometry factor (alpha),
- an order parameter of a spin probe (S), or
- a polarity surrounding a spin probe (H).

According to an embodiment, the marker linear combination for classification of a sample to be associated to a predetermined disease comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha), or
- a combination of a spectral component from free spin probe molecules (C3) and a geometry factor (alpha).

In an embodiment, the marker linear combination comprises at least one of the biophysical parameters:

- an order parameter of a spin probe (S) or
- a polarity surrounding a spin probe (H).

In an embodiment, the method is characterised in that marker linear combination comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha),
- a combination of a spectral component from free spin probe molecules (C3) and a geometry factor (alpha),
- a combination of a polarity surrounding a spin probe (H) and a rotation correlation time of a spin probe (T),
- a combination of a polarity surrounding a spin probe (H) and an order parameter of a spin probe (S),
- a combination of a polarity surrounding a spin probe (H) and a binding constant of a spin probe (KB), or
- a combination of an order parameter of a spin probe (S) and a binding constant of a spin probe (KB).

In an embodiment, marker linear combination comprises at least one of:

- a combination of a polarity surrounding a spin probe (H) and a rotation correlation time of a spin probe (T),
- a combination of a polarity surrounding a spin probe (H) and an order parameter of a spin probe (S),
- a combination of a polarity surrounding a spin probe (H) and a binding constant of a spin probe (KB), or
- a combination of an order parameter of a spin probe (S) and a binding constant of a spin probe (KB).

The marker linear combination can comprise a combination of two biophysical parameters.

In an embodiment, the marker linear combination comprises a combination of a spectral component from spin probe on lipid-fraction of serum (C5) and a spectral component from free spin probe molecules (C3). In an embodiment, the marker linear combination comprises a combination of a spectral component from spin probe on lipid-fraction of serum in solution B (C5B) and a spectral component from free spin probe molecules in solution A (C3A) and/or in solution B (C3B).

In an embodiment, the marker linear combination comprises a combination of a spectral component from spin

probe on lipid-fraction of serum (C5) and a geometry factor (alpha). In an embodiment, the marker linear combination comprises a combination of a spectral component from spin probe on lipid-fraction of serum in solution B (C5B) and a geometry factor (alpha).

In an embodiment, the marker linear combination comprises a spectral component from free spin probe molecules (C3) and a geometry factor (alpha). In an embodiment, the marker linear combination comprises a spectral component from free spin probe molecules in solution A (C3A) and/or in solution B (C3B) and a geometry factor (alpha).

In an embodiment, the marker linear combination comprises a combination of a polarity surrounding a spin probe (H) and a rotation correlation time of a spin probe (T). In an embodiment, the marker linear combination comprises a combination of a polarity surrounding a spin probe 1 in solution B (H1B) and a rotation correlation time of a spin probe 2 in solution A (T2A). In an embodiment, the marker linear combination comprises a combination of a polarity surrounding a spin probe 1 in solution B (H1B) and a ratio according to spin probe 2 in solution A (TG2A).

In an embodiment, the marker linear combination comprises a combination of a polarity surrounding a spin probe (H) and an order parameter of a spin probe (S). In an embodiment, the marker linear combination comprises a combination of a polarity surrounding a spin probe 1 in solution A (H1A) and an order parameter of a spin probe 1 in solution B (S1B).

In an embodiment, the marker linear combination comprises a combination of a polarity surrounding a spin probe (H) and a binding constant of a spin probe (KB). In an embodiment, the marker linear combination comprises a combination of a polarity surrounding a spin probe 1 in solution A (H1A) and a binding constant of a spin probe in solution B (KBB) and/or in solution C (KBC).

In an embodiment, the marker linear combination comprises a combination of an order parameter of a spin probe (S) and a binding constant of a spin probe (KB). In an embodiment, the marker linear combination comprises a combination of the order parameter of a spin probe 1 in solution B (S1B) and a binding constant of a spin probe in solution B (KBB) and/or in solution C (KBC).

The combination of biophysical parameters can improve the performance of the method. A marker linear combination including a particular combination of biophysical parameters can improve the specificity of the classification by the method according to the invention. In an embodiment, a marker linear combination including a particular combination of biophysical parameters can improve the sensitivity of the classification by the method according to the invention.

By incorporating a particular combination of two specific biophysical parameters in the marker linear combination the performance of the method is advantageously improved.

In an embodiment, the marker linear combination comprises a combination of a polarity surrounding a spin probe (H) and a rotation correlation time of a spin probe (T), in particular a combination of a polarity surrounding a spin probe, in particular a polarity surrounding a first spin probe in the solution B (H1B) and a rotation correlation time of a spin probe (T), in particular a rotation correlation time of a second spin probe in the solution A (TG2A)

According to an embodiment, the marker linear combination for classification of a sample to be associated to a stage of a hepatic disease comprises a combination of a polarity surrounding a spin probe (H) and a rotation correlation time of a spin probe (T). In particular, the marker linear combination comprising a combination of a polarity surrounding a spin probe (H) and a rotation correlation time of a spin probe (T) can be associated to a prognostic information regarding the risk of deterioration of disease. In an embodiment, the marker linear combination comprising a combination of a polarity surrounding a spin probe (H) and a rotation correlation time of a spin probe (T) is used to estimate the development of AD to ACLF. In an embodiment, the marker linear combination comprising a combination of a polarity surrounding a spin probe (H) and a ratio (TG) can be associated to a prognostic information regarding the risk of deterioration of disease, in particular to estimate the development of AD to ACLF. The marker linear combination comprising H1B and TG2A can be associated to a prognostic information regarding the risk of deterioration of disease, in particular it can be used to estimate the development of AD to ACLF.

In an embodiment, the marker linear combination comprises:

- a combination of a polarity surrounding a spin probe, in particular a polarity surrounding a first spin probe in the solution B (H1B) and a rotation correlation time of a spin probe, in particular a ratio comprising the rotation correlation time of a second spin probe in the solution A (TG2A).

Hence, for the prediction of the course of a disease, in particular, the polarity surrounding a first spin probe and a rotation correlation time of a second spin probe are included in the marker linear combination. In particular the polarity surrounding a first spin probe in the solution B is of importance. With respect to the second spin probe, its rotation correlation time in the solution A (TG2A) is included.

Hence, for the prediction of the development of the disease, in particular the development of AD, depends on the characteristics of two different spin labels (spin label 1 and spin label 2) in different environments (solution A and solution B).

In an embodiment, the marker linear combination reads:

$$k_{31}*H1B+k_{32}*TG2A \quad \text{(formula 1).}$$

In an embodiment, $k_{31}$ = 18.7, $k_{32}$ = -1.06.

According to an embodiment, the combination of the two biophysical parameters H and T outperforms the performance of the single biophysical parameter T to predict the development of AD to ACLF. In particular, the AUROC concerning the combination of H and T is greater than the AUROC related to T.

In an embodiment, the combination of the two biophysical parameters H and TG outperforms the performance of the single biophysical parameter TG, to predict the development of AD. In particular, the AUROC concerning the combination of H and TG is greater than the AUROC related to TG. In an embodiment, the combination of the two biophysical parameters H and TG outperforms the performance of the single biophysical parameter H. It outperforms the performance of individual biophysical parameters that are not included in formula 1, such as KB or DTE.

In an embodiment, the combination of the biophysical parameters H1B and TG2A outperforms the performance of TG2A, to predict the development of AD. In particular, the AUROC concerning the combination of H1B and TG2A is greater than the AUROC related to TG2A. In an embodiment, the combination of the biophysical parameters H1B and TG2A outperforms the performance of the single biophysical parameter H1B. In an embodiment, the combination of the biophysical parameters H1B and TG2A outperforms the performance of individual biophysical parameters that are not included in formula 1, such as KB or DTE. In a further embodiment, the marker linear combination comprises:

- a combination of a polarity surrounding a spin probe (H1) and a rotation correlation time of a spin probe (T), and
- a score, in particular a CLIF-C-AD score.

According to an embodiment, a score is determined. The score can be included in the marker linear combination. The marker linear combination can comprise a combination of at least two biophysical parameters and a score.

The score can be the CLIF-C-AD score. In an embodiment, the score can be one of: the MELD score, the Child-Pugh score, the MELD Na score, an indicator, such as the concentration of bilirubin, the concentration of the alanine transaminase (ALT), the concentration of the aspartate transaminase (AST) or the concentration of albumin.

In an embodiment, the prediction of the course of a disease, in particular the estimation of the development of AD to ACLF, is based on the CLIF-C-AD score and a marker linear combination comprising a combination of a polarity surrounding a spin probe (H) and a rotation correlation time of a spin probe (T).

According to an embodiment, the prediction of the course of a disease, in particular the estimation of the development of AD to ACLF, is based on the CLIF-C-AD score and a marker linear combination comprising a combination of a polarity surrounding a spin probe (H) and the ratio (TG).

By combining the CLIF-C-AD score and the marker linear combination comprising a combination of a polarity surrounding a spin probe (H) and a rotation correlation time of a spin probe (T) the performance of the method improves considerably. In an embodiment, the performance of the method is improved considerably, when combining the CLIF-C-AD score and the marker linear combination comprising a combination of a polarity surrounding a spin probe (H) and a ratio comprising the rotation correlation time of a spin probe (TG).

In an embodiment, the method is characterised in that the marker linear combination comprises a combination of a polarity surrounding a spin probe, in particular a polarity surrounding a first spin probe in the solution B (H1B) and a rotation correlation time of a spin probe, in particular a rotation correlation time of a second spin probe in the solution A (TG2A), and the CLIF-C-AD score.

In an embodiment, the method is characterised in that the marker linear combination comprises a combination of a polarity surrounding a spin probe, in particular a polarity surrounding a first spin probe in the solution B (H1B) and a ratio comprising the rotation correlation time of a spin probe, in particular a ratio comprising the rotation correlation time of a second spin probe in the solution A (TG2A), and the CLIF-C-AD score.

The combination of the biophysical parameters H1B and TG2A and the CLIF-C-AD (CLIF) score can improve the performance to estimate the development of AD to ACLF. In an embodiment, the combination of the biophysical parameters and the CLIF-C-AD score improves the performance to estimate the development of AD to ACLF in comparison to the performance of the CLIF-C-AD score alone. In an embodiment, the combination of the biophysical parameters H1B and TG2A and the CLIF-C-AD score improves the performance to estimate the development of AD to ACLF in comparison to the performance of TG2A alone. In an embodiment, the combination of two biophysical parameters and the CLIF-C-AD score improves the performance to estimate the development of AD to ACLF in comparison to the performance of the combination of H1B and TG2A.

In an embodiment, the marker linear combination reads:

$$k_{31}*H1B-k_{32}*TG2A+k_{33}*CLIF \quad \text{(formula 2).}$$

In an embodiment, $k_{31}$ = 17.8, $k_{32}$ = -0.944, $k_{33}$ =0.0842 .

According to another embodiment, the marker linear combination comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha), or
- a combination of a spectral component from free spin probe molecules (C3) and a geometry factor (alpha).

In an embodiment, the marker linear combination for classification of a sample to be associated to a predetermined disease, in particular the differentiation of patients with a compensated cirrhosis (CC) from patients with a cirrhosis with acute decompensation (AD), comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha), or
- a combination of a spectral component from free spin probe molecules (C3) and a geometry factor (alpha).

In an embodiment, the marker linear combination comprises a combination of a spectral component from spin probe on lipid-fraction of serum (C5) and a spectral component from free spin probe molecules (C3). In an embodiment, the marker linear combination comprises a combination of a spectral component from spin probe on lipid-fraction of serum in solution B (C5B) and a spectral component from free spin probe molecules in solution A (C3A) and/or in solution B (C3B).

In an embodiment, the marker linear combination comprises a combination of a spectral component from spin probe on lipid-fraction of serum (C5) and a geometry factor (alpha). In an embodiment, the marker linear combination comprises a combination of a spectral component from spin probe on lipid-fraction of serum in solution B (C5B) and a geometry factor (alpha).

In an embodiment, the marker linear combination comprises a spectral component from free spin probe molecules (C3) and a geometry factor (alpha). In an embodiment, the marker linear combination comprises a spectral component from free spin probe molecules in solution A (C3A) and/or in solution B (C3B) and a geometry factor (alpha).

In an embodiment, the marker linear combination comprises a combination of a spectral component from spin probe on lipid-fraction of serum (C5), a spectral component from free spin probe molecules (C3), and a geometry factor (alpha). In another embodiment, the marker linear combination comprises a combination of a spectral component from spin probe on lipid-fraction of serum (C5) a spectral component from free spin probe molecules (C3), a geometry factor (alpha) and a binding efficiency of a spin probe (BE).

According to an embodiment, the marker linear combination comprises at least three biophysical parameters. The marker linear combination comprising C5, C3 and alpha can outperform the performance of a marker linear combination comprising C5 and alpha to classify samples, in particular to distinguish between AD and CC samples. The marker linear combination comprising C5, C3 and alpha can outperform the performance of a marker linear combination comprising C3 and alpha. The marker linear combination comprising C5, C3 and alpha can outperform the performance of each individual biophysical parameters, in particular C3, C5 or alpha. In particular, the marker linear combination comprising C5, C3 and alpha can outperform the performance of each individual biophysical parameters, in particular C3, C5 or alpha, to discriminate samples associated to AD patients and samples associated to CC patients.

According to an embodiment, the marker linear combination comprising C5, C3, BE and alpha can outperform the performance of each individual biophysical parameters, in particular C3, C5, BE or alpha, to discriminate samples associated to AD patients and samples associated to CC patients.

According to an embodiment, the marker linear combination comprises a combination of a binding efficiency of a spin probe (BE), a geometry factor (alpha), a spectral component from free spin probe molecules, in particular a spectral component from free spin probe molecules in the solution A (C3A) and a spectral component from free spin probe molecules in the solution B (C3B), and a spectral component from spin probe on lipid-fraction of serum, in particular a spectral component from spin probe on lipid-fraction of serum in the solution B (C5B).

According to an embodiment, the marker linear combination reads:

$$k_{21}*BE+k_{22}*Alpha+k_{23}*C3A+k_{24}*C3B+k_{25}*C5B \quad \text{(formula 3)}$$

In an embodiment, $k_{21}$ = -5.84; $k_{22}$= 9.63; $k_{23}$= -775; $k_{24}$= -230, $k_{25}$ = -135.

Each of the combinations C3 and C5, C3 and alpha, and C5 and alpha can outperform the performance of a single biophysical parameter C3, C5, alpha, particularly to classify a sample to be associated to AD or CC. In particular, for each combination C3 and C5, C3 and alpha, or C5 and alpha, the AUROC related to the respective combination can be greater than the AUROC related to the individual biophysical parameter, in particular greater than the AUROC related to alpha, C3 or C5. Further, each combination C3 and C5, C3 and alpha, or C5 and alpha can outperform the performance of a single biophysical parameter that differs from alpha, C3 and C5. In an embodiment, a combination of C3 and C5, C3 and alpha, or C5 and alpha outperforms the performance of a combination of two biophysical parameters differing from C3 and C5, C3 and alpha, or C5 and alpha. Hence, by considering a combination of two biophysical parameters, in particular C3 and C5, C3 and alpha, and/or C5 and alpha, the performance of the method is advantageously improved.

The marker linear combination according to formula 3 can outperform the performance of the individual biophysical parameters to classify a sample to be associated to AD or CC. In an embodiment, the marker linear combination comprising BE, alpha, C3A, C3B and C5B outperforms the performance of a combination of C3 and C5, C3 and alpha, and C5 and alpha to classify a sample to be associated to AD or CC.

In an embodiment, the marker linear combination comprises at least one of:

- a combination of a polarity surrounding a spin probe (H) and an order parameter of a spin probe (S),
- a combination of a polarity surrounding a spin probe (H) and a binding constant of a spin probe (KB), or
- a combination of an order parameter of a spin probe (S) and a binding constant of a spin probe (KB).

In an embodiment, the marker linear combination for classification of a sample to be associated to a predetermined disease, in particular the differentiation of patients with a cirrhosis with acute decompensation (AD) from patients with an acute on chronic liver failure (ACLF) comprises at least one of:

- a combination of a polarity surrounding a spin probe (H) and an order parameter of a spin probe (S),
- a combination of a polarity surrounding a spin probe (H) and a binding constant of a spin probe (KB), or
- a combination of an order parameter of a spin probe (S) and a binding constant of a spin probe (KB).

In an embodiment, the marker linear combination comprises a combination of a polarity surrounding a spin probe (H) and an order parameter of a spin probe (S). In an embodiment, the marker linear combination comprises a combination of a polarity surrounding a spin probe 1 in solution A (H1A) and an order parameter of a spin probe 1 in solution B (S1B).

In an embodiment, the marker linear combination comprises a combination of a polarity surrounding a spin probe (H) and a binding constant of a spin probe (KB). In an embodiment, the marker linear combination comprises a combination of a polarity surrounding a spin probe 1 in solution A (H1A) and a binding constant of a spin probe in solution B (KBB) and/or in solution C (KBC).

In an embodiment, the marker linear combination comprises a combination of an order parameter of a spin probe (S) and a binding constant of a spin probe (KB). In an embodiment, the marker linear combination comprises a combination of the order parameter of a spin probe 1 in solution B (S1B) and a binding constant of a spin probe in solution B (KBB) and/or in solution C (KBC).

The marker linear combination comprising H1A, KBB and KBC outperforms the performance of single individual biophysical parameters. The marker linear combination outperforms the performance of individual biophysical parameters of the marker linear combination, such as H1A, in particular to classify samples to be associated to AD or ACLF.

An embodiment is characterised in that the marker linear combination comprises a combination of a polarity surrounding a spin probe (H) and an order parameter of a spin probe (S), and a binding constant of a spin probe (KB).

In an embodiment, the marker linear combination comprises a combination of a polarity surrounding a spin probe 1 in solution A (H1A), an order parameter of a spin probe 1 in solution B (S1B), and a binding constant of a spin probe in solution B (KBB) and/or in solution C (KBC).

The marker linear combination comprising H1A, S1B, KBB and KBC outperforms the performance of the marker linear combination comprising H1A, KBB and KBC. In particular, the marker linear combination comprising H1A, S1B, KBB and KBC outperforms the performance of the marker linear combination comprising H1A, KBB and KBC to classify a sample to be associated to AD or ACLF.

In an embodiment, the marker linear combination comprises:

- a combination of a polarity surrounding a spin probe (H), an order parameter of a spin probe (S) and a binding constant of a spin probe (KB),

and at least one of:

- a binding efficiency of a spin probe (BE),
- a detoxification efficiency (DTE),
- a spectral component from spin probe on lipid-fraction of serum (C5), or
- a rotation correlation time of a spin probe (T).

In an embodiment, the marker linear combination comprises:

- a combination of a polarity surrounding a spin probe (H), an order parameter of a spin probe (S) and a binding constant of a spin probe (KB),

and at least one of:

- a binding efficiency of a spin probe (BE),
- a detoxification efficiency (DTE),
- a spectral component from spin probe on lipid-fraction of serum (C5), or
- a ratio comprising a rotation correlation time of a spin probe (TG).

According to an embodiment, the marker linear combination comprises H, KB, S and the binding efficiency of a spin probe (BE).

In a further embodiment, the marker linear combination comprises H, KB, S and the detoxification efficiency (DTE).

In an embodiment, the marker linear combination comprises H, KB, S and the spectral component from spin probe on lipid-fraction of serum (C5).

In an embodiment, the marker linear combination comprises H, KB, S and the rotation correlation time of a spin probe (T).

In an embodiment, the marker linear combination comprises H, KB, S and the ratio comprising the rotation correlation time of a spin probe (TG).

According to a further embodiment, the marker linear combination comprises:

- a combination of a binding efficiency of a spin probe (BE), a detoxification efficiency (DTE), a binding constant of a spin probe, in particular of a binding constant of a spin probe in a solution B (KBB) and a binding constant of a spin probe in a solution C (KBC), a spectral component from spin probe on lipid-fraction of serum, in particular a spectral component from spin probe on lipid-fraction of serum in the solution B (C5B), a polarity surrounding a spin probe, in particular a polarity surrounding a first spin probe in a solution A (H1A), an order parameter of a spin probe, in particular an order parameter of the first spin probe in the solution B (S1B), and a rotation correlation time of a spin probe, in particular a rotation correlation time of a second spin probe in the solution A (TG2A).

In an embodiment, the marker linear combination reads:

$$k_{11}*BE+k_{12}*DTE+k_{13}*KBB+k_{14}*KBC+k_{15}*C5B+k_{16}*H1A+k_{17}*S1B+k_{18}*TG2A,$$

(formula 4).

In an embodiment, $k_{11}$ =25.4, $k_{12}$= -27.9, $k_{13}$=0.215, $k_{14}$= -0.386, $k_{15}$=-89.2, $k_{16}$=14.7, $k_{17}$=-147, $k_{18}$= -0.524.

In an embodiment, the marker linear combination comprising BE, DTE, KBB, KBC, C5B, H1A, S1B and TG2A outperforms the performance of the marker linear combination comprising H1A, KBB and KBC. In particular, the marker linear combination comprising BE, DTE, KBB, KBC, C5B, H1A, S1B and TG2A outperforms the performance of the marker linear combination comprising H1A, KBB and KBC to classify a sample to be associated to AD or ACLF. The marker linear combination comprising BE, DTE, KBB, KBC, C5B, H1A,

S1B and TG2A can outperform the performance of the performance of the individual biophysical parameters comprised in the marker linear combination to classify a sample to be associated to AD or ACLF.

In an embodiment, the predetermined disease is a hepatic disease. In an embodiment, the predetermined disease is a stage of a hepatic disease. According to an embodiment, the predetermined disease is a predefined hepatic function, in particular an abnormal hepatic function. In particular, the predetermined disease is one of: a compensated cirrhosis (CC), a cirrhosis with acute decompensation (AD), an acute on chronic liver failure (ACLF), an insensitivity with respect to a therapy, a prognostic information regarding a risk of death in a certain time frame or a risk of deterioration of a disease.

Within the context of the application, a hepatic disease is also referred to as liver failure.

For instance, for the evolvement of AD to ACLF, the risk of deterioration of a disease is of importance. Besides a probability to develop an ACLF, also the time range of the evolvement is of diagnostic interest, in particular to advise the best therapy.

The different hepatic diseases require a treatment, matched with the particular disease. Hence, not only the classification of a liver failure is important but the classification of the particular disease to provide the particular treatment.

In an embodiment, the method is configured to detect the disease in an early stage. In consequence, a treatment of the disease at a very early stage can be provided. This can increase the possibility of an effective treatment of the disease.

In an embodiment, a cut-off related to the marker linear combination is set, wherein the cut-off is set such that when the value related to the marker linear combination is equal or greater than the cut-off, the related sample is classified to be associated to a hepatic disease, a stage of a hepatic disease and/or a predefined hepatic function.

The cut-off can be chosen according to a predefined (chosen) specificity. In an embodiment, the cut-off is chosen according to a predefined sensitivity. The cut-off can be chosen according to the particular medical issue in question.

A further aspect of the invention is related to a method for initial training of a logistic regression model or a linear model for multivariate data. The method comprises the steps of:

- providing a first plurality of spectra acquired from a corresponding plurality of aliquots containing a biophysiological carrier protein, wherein at least one of a concentration of a spin probe and a concentration of a polar reagent varies between the aliquots, wherein the first plurality of spectra is related to a first sample,

- providing a second plurality of spectra acquired from a corresponding plurality of aliquots containing a biophysiological carrier protein, wherein at least one of a concentration of a spin probe and a concentration of a polar reagent varies between the aliquots, wherein the second plurality of spectra is related to a second sample,
  wherein the first sample and the second sample differ in a predetermined disease,

- determining a first set of biophysical parameters, wherein the first set of biophysical parameters is related to the first plurality of spectra,

- determining a second set of biophysical parameters, wherein the second set of biophysical parameters is related to the second plurality of spectra, and

- providing at least a subset of the first set of biophysical parameters and at least a subset of the second set of biophysical parameters to a logistic regression model or a linear model for multivariate data to determine a marker linear combination exhibiting the largest variation between the first sample and the second sample.

The plurality of spectra can comprise a plurality of ESR spectra.

The first sample can be associated to a healthy patient. In a further embodiment, the first sample is associated

to a first liver failure. The first liver failure can be a CC, an AD or an ACLF.

The second sample can be associated to a second liver failure. The second liver failure can be a CC, an AD or an ACLF.

The first sample and the second sample differ in at least one of a hepatic disease, a stage of a hepatic disease or a predefined hepatic function.

A linear model for multivariate data can be a principle component analysis (PCA).

In an embodiment, after determining the first set of biophysical parameters and determining the second set of biophysical parameters, the first set of biophysical parameters and the second set of biophysical parameters are compared, a set of selected biophysical parameters that differ between the first set of biophysical parameters and the second set of biophysical parameters is selected and the set of selected biophysical parameters is provided to the logistic regression model or the linear model for multivariate data to determine a marker linear combination exhibiting the largest variation between the first sample and the second sample.

In this method, the most informative biophysical parameters are preselected before used for model training. This accelerates the training of the logistic regression model or the linear model for multivariate data.

A further aspect of the invention is related to a non-transitory storage medium storing program instructions for execution by a processor, the program instructions configured to, when executed by the processor, cause the processor to perform a method according the invention.

Another aspect of the invention is related to an analysis system comprising a processor. The analysis system comprises memory storing program instructions suitable for execution by said processor and a trained logistic regression model or a linear model for multivariate data, the logistic regression model or the linear model for multivariate data trained to determine a probability of applied input parameters relating to one or more of a plurality of predetermined diseases. The analysis system further comprises an input interface for receiving spectral data; and an output interface for outputting a computation result. The program instructions are configured to cause the processor, when executed by the processor:

receive a plurality of spectra acquired from aliquots containing a biophysiological carrier protein, wherein at least one of a concentration of a spin probe and a concentration of a polar reagent varies between the aliquots;
determine biophysical parameters based on the received spectra;
applying by the computing device the biophysical parameters as an input to a marker linear combination, wherein the marker linear combination is determined by a predefined combination of at least two biophysical parameters,
use the marker linear combination to determine the input parameters relating to one or more of said predetermined diseases, and
outputting a result according to the marker linear combination.

In the following, further features, advantages and embodiments of the present invention are explained with reference to the Figures, wherein

- Fig. 1 shows a schematic diagram of the structure of human serum albumin for use in an embodiment,
- Fig. 2 shows a 9.45 GHz ESR spectrum of human serum albumin,
- Fig. 3 shows five sub-spectra of the spectrum shown in Fig. 5, i.e. five spectral components,
- Fig. 4 shows an example of an acquired ESR spectrum, illustrating the relation between an ESR spectrum and the geometry factor alpha,
- Fig. 5 shows a system of an embodiment comprising an ESR spectrometer and a computing device,
- Fig. 6 shows the albumin concentration (A) and the detoxification efficiency (DTE) (B) for control, CC (stable), AD and ACLF samples,
- Fig. 7 shows the binding efficiency (BE) for control, CC (stable), AD and ACLF samples, as a dot plot (A, C) and a box plot (B),
- Fig. 8 shows values according to the marker linear combination according to formula (3) for CC (stable) samples and AD samples,
- Fig. 9 shows the ROC curves (A) and comparison of AUROC (B) according to BE, DTE and marker linear combination according to formula 3,
- Fig. 10 shows the ROC curves (A) and comparison of AUROC (B) according to BE, DTE and marker linear combination according to formula 4,
- Fig. 11 shows the ROC curves (A, B) and comparison of AUROC (C) according to BE, DTE, CLIF-C-AD score and marker linear combinations according to formula 1 and 2,
- Fig. 12 shows a Kaplan-Meier survival curve of AD and ACLF patients,
- Fig. 13 shows ROC curves related to H, combination of H and KB, combination of H, KB and S, and marker linear combination according to formula 4,
- Fig. 14 shows ROC curves related to alpha, combination of alpha and C5, combination of alpha, C5 and C3, and

marker linear combination according to formula 3 (A) and ROC curves related to C5, combination of alpha and C5, and combination of alpha, C5 and C3 (B),

Fig. 15 shows ROC curves related to alpha, combination of alpha and C5, combination of alpha, C5 and C3, and marker linear combination according to formula 3, for patients with bacterial infection, and

Fig. 16 shows ROC curves related to T, CLIF-C-AD, combination of H and T, and combination of H, T and CLIF-C-AD score.

Fig. 1 is a schematic diagram of the structure of human serum albumin (HSA) 1 for use in an embodiment. The structure of HSA 1 comprises three homologous domains 10, 20, 30 that fold into a heart-shaped albumin molecule 1. X-ray crystallographic analysis of HSA has identified at least seven distinct fatty acid binding sites (FA1-FA7) that are located in various parts of the protein (Bhattacharya et al. J Mol Biol. 2000 Nov 10;303(5):721-32) and which show relative affinities for fatty acids (Simard et al. J Mol Biol. 2006 Aug 11;361(2):336-51). The FA binding sites have certain common features: in each case, the hydrocarbon chain of the fatty acid is accommodated in a long and narrow hydrophobic well, while the carboxyl moiety is located near basic or polar residues (Curry et al. Nat Struct Biol. 1998 Sep;5(9):827-35). These FA binding sites (FA1-FA7) represent specific (primary) hydrophobic binding sites for fatty acids in HSA. In addition, non-specific (secondary) hydrophobic binding sites for fatty acids on serum albumin have been identified. These non-specific hydrophobic binding sites are believed to be located in the hydrophobic area 40 between the albumin domains 10, 20, 30 (Gurachevsky et al. Biochem Biophys Res Commun. 2007 Sep 7;360(4):852-6). It is believed that there is migration of fatty acids between the non-specific binding sites in the hydrophobic area 40 of HSA and the specific FA binding sites (FA1-FA7) and vice versa and that this process is important for the transport properties of albumin.

Albumin is produced in the liver. In case of a hepatic disease, the albumin concentration is reduced. Further, albumin can have an abnormal functionality in case of a hepatic disease, for instance, its ability to bind a particular ligand can be altered.

The properties of albumin to bind a ligand can be determined by means of a plurality of ESR spectra, in particular be means of at least one characteristic biophysical parameter derived from the plurality of ESR spectra. This means that by means of a plurality of ESR spectra, a binding property of albumin can be determined that can be an indication of a particular hepatic disease.

The procedure of the evaluation of albumin by ESR spectroscopy is also described in international patent application WO 01/65270, the entirety of which is incorporated herein by reference and comprises the following steps: In a first step, a sample aliquot containing albumin is placed into a container. In one embodiment, the sample is a serum sample. In one embodiment, the sample is a blood sample or a drug or product that contains albumin. For example, in one embodiment, the sample is a commercial solution comprising a human or bovine albumin preparation. In one embodiment, three sample aliquots are used. However, in an alternative embodiment, fewer or more sample aliquots are used; for example, 1, 2, 4, 5, 6, 7 or 8. In one embodiment, prior to step (1), a pre-analytical phase is conducted in order to preserve the original (native) conformational state of albumin as it is comprised within the sample to be evaluated. It is preferred that one or more of the following considerations are taken into account.

It is preferred that serum and EDTA-plasma samples are used. It is preferred that preparations with preservatives or anticoagulants (such as heparin), which can bind albumin and modify its native conformational state, are avoided. In embodiments where the sample is whole blood, it is preferred that the process of haemolysis is avoided. In one embodiment, to reduce haemolysis, centrifugation of whole blood for serum sampling is done within one hour of sampling at room temperature. In one embodiment, centrifugation is performed for 10 minutes at 1000 to 1500 g. In one embodiment, vacuum sampling systems are avoided during sampling of whole blood as it is believed that this can influence the stability of certain whole blood and serum samples over time. It is preferred that freezing of the whole blood sample before serum sampling is avoided as it is believed that this disturbs the native conformational state of the components of whole blood. In one embodiment, whole blood is stored or transported cooled for a maximum of 24 hours before centrifugation. In one embodiment, separated serum or EDTA-plasma is stored before analysis in a frozen state at a temperature not higher than -28°C (due to on-going biochemical prepossess that are believed to occur even in frozen serum). It is preferred that samples are unfrozen only once and that this is shortly before use in the procedure. In one embodiment, the maximum time between defrosting the sample and measurement in the ESR spectrometer is 40 minutes. In embodiments where the sample is an albumin containing preparation such as a commercial albumin solution or a control sample, the recommendations of the manufacturer regarding the preparation of a control sample, an in particular the dilution of lyophilised albumin, are considered. It is preferred to have enough material of each sample for at least one controlling repetition of the measurement. Furthermore, it is preferred that samples from donors that fall within certain categories are excluded. In one embodiment, these categories comprise patient less than 21 days post surgery of a defined invasive procedure, as defined in the NCI dictionary (https://www.cancer.gov/publications/dictionaries/cancer-terms/def/invasive-procedure).

Secondly, each sample aliquot is mixed with a spin probe in a polar reagent. A spin probe (also known as a spin label) is an organic molecule that possesses an unpaired electron and has the ability to bind to another molecule.

In the present embodiment, the spin probe is 16-doxyl stearic acid. In an alternative embodiment, the spin probe is an alternative spin-labelled fatty acid, preferably a doxyl stearic acid, and is one of 5-, 7-, 12- or 16-doxyl stearic acid or 16-doxyl stearate (Soduim). In one embodiment, it is a doxyl lauric acid, preferably 7-doxyl lauric acid. In one embodiment, any spin-labelled compound which can undergo specific binding to albumin (including a further spin-labelled fatty acid, a steroid hormone or a heterocyclic hydrocarbon) is used as a spin probe. Hydrophobic compounds labelled with nitroxyl radicals are used in one embodiment. In the present embodiment, the polar reagent is ethanol. In an alternative embodiment, an alternative alcohol or DMSO is used. It is preferred to use a C1-C6 alcohol. In the present embodiment, the polar reagent acts as a solvent for the spin probe and acts to modify the polarity of the mixture. As will be described in further detail below, in the present embodiment, the three sample aliquots vary in the concentration of albumin and spin probe. In addition, the strength of hydrophobic interactions in the albumin-spin probe mixture is varied through use of differing amounts of polar reagent. In alternative embodiments, fewer or more than three different concentrations of albumin, spin probe and/or polar reagent are used; for example, 1, 2, 4, 5, 6, 7 or 8. Varying the concentration of spin label that is added to albumin and changing the ionic strength of the spin label mixture enables ESR spectra to be generated under different conditions. Suitable spin probes and polar reagents are known and some suitable spin probes and polar reagents are disclosed in WO 01/65270, the entirety of which is incorporated herein by this reference.

In an embodiment, three different concentrations of spin probe of 3.5, 5.8 and 7.5 mmol/l are mixed with 50 $\mu$l of each serum sample aliquot in the volumes of 10, 12 and 14 $\mu$l respectively. In one embodiment, the mean value of the ratio of spin probe concentration to albumin concentration is 2.5 $\pm$ 0.5 and, starting from this mean value, at least two additional concentrations are selected whose deviation from this mean value is no less than 1.0. The concentrations of polar reagent to be added are selected in such a way that the mean value of the final concentration of polar reagent in the aliquots is (0.6 $\pm$ 0.25)xCp, wherein Cp represents the critical concentration of polar reagent, surpassing of which would result in denaturing of the albumin, and, starting from this mean value, at least two additional concentrations of polar reagent are selected, whose deviation from this mean value is at least 15%. Further details on the proportions of spin probe, albumin and polar reagent are described in US 2003/170912 A1/US Patent No. 7,166,474, which are incorporated herein by reference in their entirety. Without wishing to be bound by theory, by varying the concentration of spin probe and the concentration of polar reagent, it is believed that a combination of concentrations can be produced, which enables the transport properties of the albumin to be detected at different stages, namely, the physiological state during binding of hydrophobic compounds such as fatty acids (low concentration of spin probe and low concentration of polar reagent), the physiological state during transport of hydrophobic compounds through the vascular system (high concentration of spin probe and low concentration of polar reagent), and the physiological state during delivery (release) of hydrophobic compounds to the target cells (high concentration of spin probe and high concentration of polar reagent).

In a third step of the exemplary procedure, the mixture of the sample, spin probe and polar reagent is incubated. In the present embodiment, the incubation period is 10 minutes at 37°C and at the physiological pH of blood. In an alternative embodiment, the incubation period is less or more than 10 minutes; for example, from 7 to 15 minutes. In an alternative embodiment, two or more different temperature values of the samples ranging between 15 and 45°C and/or two or more different pH values of the serum samples ranging from 7.5 to 3.5 are used. In a following step, the mixture can be taken up by capillary tubes. In a subsequent step, an ESR spectrometer can be used to measure ESR spectra from each capillary tube. In a subsequent step, the ESR spectra can be analysed and results can be calculated.

To perform ESR spectroscopy the capillary tube can be inserted into an ESR spectrometer. Suitable ESR spectrometers are available from MedInnovation GmbH (Berlin, Germany), for example models EPR 01-08, MS-400 and Espire-5000. ESR spectroscopy is a known technique that does not need to be discussed in detail in the present disclosure. Briefly, however, ESR spectra are acquired by exposing the sample to a strong static magnetic field. The application of the static magnetic field causes the separation of free electrons into two spin states. The application of microwave energy at the correct frequency causes spins to transition between the states. The microwave energy absorbed in this transition is measurable. It is either possible to maintain the microwave frequency constant and change the strength of the static magnetic field while monitoring the amount of microwave energy that is being absorbed or keeping the static magnetic field unchanged while sweeping the microware frequency over a range whilst monitoring energy absorption. The exact static magnetic field strength/microwave frequency combination that provides the correct amount of energy required by a spin to transition between the spin states depends on the chemical environment of the spin. It will be understood that a given spin probe consequently requires different amounts of energy to accomplish this transition, depending on the whether or not the spin probe is bound to albumin and indeed on the manner in which it is bound to albumin. Consequently ESR based methods can distinguish between unbound and bound spin probes. Moreover, ESR is able to distinguish between spin probes located at different binding sites on the albumin complex or between different spin probes in different unbound conditions. As such ESR is a powerful tool for assessing the binding conditions found on a molecule, in the embodiment on serum albumin.

An EPR spectrum can be generated by tracking the amount of microwave energy absorbed as the strength of the static magnetic field is ramped up or down over a predetermined range and by forming the first derivative of the tracked absorption spectrum. As the strength of the static magnetic field changes, so does the separation between the

two spin states of the free electron. The separation between these two spin states does not only depend on the applied static magnetic field strength but also on the chemical environment within which the spin is located. By observing the amount of energy absorbed at each given static magnetic field strength conclusions can be drawn regarding the amount of spin probe present in a binding state that produces a separation in the energy.

Other X-Band EPR spectrometers (operating with a microwave frequency of approximately 9-10 GHz, can be also used in embodiments. The sample can be maintained at 37°C during the measurement process to mimic physiologic conditions. Particular binding sites of spin probes on albumin produce spectral ESR patterns all signatures that can be detected. Analysis of such signatures allows determination of the amount of spin probe bound to the various binding sites. This said, it is not unusual for ESR patterns are generated by the influence different binding sites have on spin probes to overlap with each other.

Fig. 2 illustrates a 9.45 GHz ESR spectrum of human serum albumin. This spectrum consists of a number of overlapping sub-spectra. The measured spectrum shown in Fig. 2 can, for example, be decomposed into five sub-spectra, as shown in Fig. 3. The spectral components mentioned in Fig. 3 described in Table 1 and relate to spin probes respectively bound to albumin with high and low affinity or are present in the serum in the states mentioned in the table (Table 1).

Table 1: Spectral components

| | |
|---|---|
| C1 | low motion albumin bound component with a high affinity |
| C2 | low motion albumin bound component with a lower affinity |
| C3 | free spin probe (16-doxyl stearic acid) molecules |
| C4 | free spin probe (16-doxyl stearic acid) in micelles |
| C5 | spin probe (16-doxyl stearic acid) bound on lipid-fraction of the serum |

C1 represents the proportion of the fatty acid spin probe bound to the specific binding sites in albumin (i.e. at FA binding sites FA2, FA4 and FA5). C2 represents the proportion of fatty acid spin probe bound to the non-specific binding sites, binding sites 1, 3, 6 and 7 in the hydrophobic area of albumin. C3 to C5 represent unbound spin probe molecules (i.e. spin probes that are not bound to albumin). More specifically, C3 represents the proportion of spin probe molecules that are unbound and present free in the sample; C4 represents the proportion of spin probe molecules that are aggregated into clusters of fatty acid micelles; and C5 represents the proportion of spin probe molecules that are associated with or bound to lipoproteins in the serum sample. The contributions individual spectral components make to the measure spectrum can be determined by simulating the individual spectral components and then fitting the simulated components to the measured spectrum, adjusting the magnitude and phase of the individual spectral components until a fitting criterion is fulfilled. This criterion may, for example, be the minimisation of the root mean square error between the measured spectrum and the sum of the individual simulated spectral components as weighted by the above mentioned phase and magnitude term. The magnitudes and phases the spectral components have after this fitting indicate the concentration of spin probes in the various binding states set out in Table 1. In an embodiment the spectral components are simulated in the manner described by Andrey Gurachevsky, Ekaterina Shimanovitch, Tatjana Gurachevskaya, Vladimir Muravsky (2007) Intra-albumin migration of bound fatty acid probed by spin label ESR. Biochemical and Biophysical Research Communications 360 (2007) 852-856, the entirety of which is incorporated herein by this reference. The details of the method of simulation the spectral components need not be discussed in detail in the present disclosure. In an embodiment the simulated spectral lines are fitted to the measured ESR spectrum using a least squares fit. Alternatively, maximum likelihood estimation may be used. Based on the fitting of the five simulated components C1 to C5 as defined in Table 1, the relative concentrations of components C1 to C5 for each of the three above described aliquots (hereinafter also referred to by the short-hand A, B, C) are determined. This can be done in the manner described in WO 2000/004387A3, the entirety of which is incorporated herein by reference.

Fig. 4 illustrates the relation between an ESR spectrum and the geometry factor alpha. Fig. 4 shows an ESR spectrum, A, B or C. The geometry factor alpha is calculated as the ratio of the amplitude of the left-most spectral component of the ESR spectrum and the magnitude of the last but one spectral component on the right hand side of the ESR spectrum. These two peaks are related by their g-factors. Alpha is the ratio of intensities at the position of the high-field line of unbound 16-doxyl-stearic spin label relative to the spectral intensity at the position of low-field line of the bound 16- DSA in the albumin binding sites with high affinity. Fig. 4 illustrates that the geometry factor is determined by the raw spectra, such that it is not dependent on particular assumptions and/or simplifications of the model for simulation of EPR spectra.

Fig. 5 depicts a system comprising an ESR spectrometer 10 and a computing device 12. The ESR spectrometer comprises an experimental section in which spectra are acquired in the manner discussed above from samples entered

into a sample chamber. Acquired spectra are output to the computing device 12 via the output interface 16 of the ESR spectrometer and the input interface 18 of the computing device 12. The computing device 12 comprises a processor 20 and a memory 22. The memory 22 stores program instructions for execution by the processor 20. The program instruction cause the processor 20 to perform the methods described herein when executed by the processor 20. The computing device 12 further comprises an output device 24. The output device 24 may be a display for displaying a determination result to the user or may be an electronic output interface that allows results to be transmitted to other devices. Any such transmission may take place using wireless or wired means.

Data associated to healthy patients (control), CC (stable), AD and ACLF patients is analysed by means of an embodiment of the method. Data of 10 control samples, 18 stable samples (outpatients, CC), 241 AD samples and 78 ACLF samples is considered. Further information regarding CC (outpatient, stable), AD and ACLF patients, including demographic, biochemical and clinical characteristics, is provided in Table 2.

Table 2: demographic, biochemical and clinical characteristics of patients. Demographic data comprises age and sex. The aetiology of cirrhosis is characterised in terms of a viral, alcoholic, NASH (non-alcoholic steatohepatitis), a mixed aetiology or other causes. Biochemical and prognostic data comprises number of leucocytes, number of platelets, concentration of Na, concentration of bilirubin, concentration of creatinine, INR, MELD score, and Child-Pugh score. Data is reported as median and inter quartile range (IQR), absolute number and frequency. P-values indicate significance. Significant p-values are indicated in bold.

| | **Outpatients** | **AD** | **ACLF** | **p** |
|---|---|---|---|---|
| N | 18 | 241 | 78 | |
| **Demographic data** | | | | |
| Age (years) | 59 (53-77) | 63 (51-75) | 62 (56-74) | 0.800 |
| Male sex | 15 (83) | 144 (60) | 51 (65) | 0.112 |
| **Etiology of cirrhosis** | | | | |
| Viral | 8 (44) | 118 (49) | 28 (36) | 0.131 |
| Alcohol | 5 (28) | 41 (17) | 17 (22) | 0.383 |
| NASH | 0 (0) | 17 (7) | 7 (9) | 0.409 |
| Mixed etiology | 0 (0) | 35 (15) | 10 (13) | 0.215 |
| Other | 5 (28) | 30 (12) | 16 (21) | 0.069 |
| **Biochemical and prognostic data** | | | | |
| Leucocyte ($10^9$/L) | 5.0 (3.8-6.7) | 5.5 (3.6-8.5) | 7.5 (5.1-9.6) | **0.006** |
| Platelets ($10^9$/L) | 139 (101-159) | 95 (59-158) | 86 (55-144) | 0.102 |
| Sodium (mmol/L) | 141 (138-142) | 137 (134-139) | 136 (132-139) | **0.001** |
| Bilirubin (mg/dL) | 0.9 (0.5-1.4) | 2.2 (1.1-3.7) | 6.8 (2.1-14.5) | **<0.001** |
| Creatinine (mg/dL) | 0.8 (0.7-0.9) | 0.9 (0.7-1.2) | 1.9 (1.4-2.5) | **<0.001** |
| INR | 1.1 (1.1-1.3) | 1.4 (1.2-1.5) | 1.6 (1.3-2.2) | **<0.001** |
| MELD | 13 (10-17) | 14 (10-17) | 26 (22-30) | **<0.001** |
| Child-Pugh score | 6 (5-6) | 8 (7-10) | 11 (10-12) | **<0.001** |

Fig. 6A gives the albumin concentration related to samples of healthy patients (control), CC (stable), AD and ACLF patients as box-and-whisker plots. In the box-and-whisker plot, the central box represents the values from the lower to upper quartile (25 to 75 percentile). Hence, the box represents the IQR. The middle line represents the median. The horizontal line extends from the minimum to the maximum value, excluding outside and far out values, which are displayed as separate points. The median albumin concentration is highest in control samples and lowest in ACLF patients. The median albumin concentration in AD samples differs significantly with the median albumin concentration of control samples as well as with the median albumin concentration of samples of CC patients. However, the median albumin concentration of AD patients does not differ significantly with median albumin concentration associated to ACLF samples. The IQR related to AD samples overlap with the IQR related to the ACLF sample. Taken together, based on the albumin concentration a differentiation between AD and ACLF samples is impossible.

Fig. 6B gives the detoxification efficiency (DTE) related to samples of healthy patients (control), CC (stable), AD and ACLF patients as box-and-whisker plots. The DTE in % of the control is given. The DTE in control sample is highest, the DTE in ACLF is lowest. The DTE of AD samples differs significantly with DTE in control, CC (stable) as well as ACLF. The IQR related to AD samples overlap with the IQR related to the ACLF sample.

Fig. 7 gives the binding efficiency (BE) related to samples of healthy patients (control), CC (stable), AD and ACLF patients as dot plots (A) and box-and-whisker plots (B). The BE in % of the control is given. The BE in control sample is highest, the BE in ACLF is lowest. The BE of AD samples differs significantly with BE in control, CC (stable) as well as ACLF. The IQR related to AD samples overlap with the IQR related to the ACLF sample.

In Fig. 7C, data related to AD and CC (stable) patients is shown as dot plots, wherein additionally a cut-off (straight line) is shown. The cut-off is set such that a sensitivity of about 92% is set. At a sensitivity of 91.7%, a specificity of 61.1% is reached.

In Fig. 8A, marker linear combination according to formula 3 related to AD and CC (stable) patients is shown as dot plots. Median values are indicated as lines. In Fig. 8B, the data shown in Fig. 8A is presented and a cut-off which is set such that the cut-off is set such that a sensitivity of about 92% is set. At a sensitivity of 92.4%, a specificity of 83.3% is reached. Comparing the specificity according to BE (Fig. 7C) and marker linear combination according to formula 3 (Fig. 8B) at about 92%, the specificity of marker linear combination according to formula 3 is increased considerably. In comparison to the specificity related to BE, the specificity according to the marker linear combination according to formula 3 is increased about 22.2%. This means that about 22% more patients are classified correctly as CC patients.

Fig. 8C exemplifies a cut-off set at a specificity of 100%. At 100% specificity, the marker linear combination according to formula 3 has a sensitivity of 68.1%. A specificity of 100% implies that all CC (stable) patients are classified correctly as CC (stable) patients.

For further investigations, the ROC curves are calculated and analysed. In particular, the respective AUROC is determined and compared.

For comparison of CC and AD patients, the ROC curves associated to BE, DTE and marker linear combination according to formula 3 ("combination 1") are shown in Fig. 9A. In Fig. 9B, the respective AUROC values are given, in particular the median. The 95% confidence interval is provided in brackets. Further, the p-values are given. A p-value is a measure for the significance. Typically with a p-value smaller than 0.05, significance is accepted. The closer the AUROC is to one, the better the performance.

The AUROC related to the marker linear combination according to formula 3 is 0.94 (0.9 - 0.96), while the AUROC related to BE is 0.87 (0.82 - 0.91) and the AUROC related to DTE is 0.85 (0.8 - 0.89). Hence, the AUROC related to the marker linear combination according to formula 3 is greater than the AUROC related to BE and is greater than the AUROC related to DTE. The improvement is significant (p=0.01). This means that the performance of the marker linear combination according to formula 3 (to distinguish between AD and CC samples) is increased in comparison to the performance of BE alone or DTE alone.

For comparison of AD patients and ACLF patients, the ROC curves associated to BE, DTE and marker linear combination according to formula 4 ("combination 2") are shown in Fig. 10A. In Fig. 10B, the respective AUROC values are given as well as p-values.

The AUROC related to the marker linear combination according to formula 4 is 0.72 (0.67 - 0.77), while the AUROC related to BE is 0.64 (0.59 - 0.7) and the AUROC related to DTE is 0.66 (0.6 - 0.71). Hence, the median AUROC related to the marker linear combination according to formula 4 is greater than the median AUROC related to BE and is greater than the median AUROC related to DTE. The increase is partially significant. The performance of the marker linear combination according to formula 4 is increased in comparison to the performance of BE alone or DTE alone.

For prognostic information about the development of AD, in particular the characterisation of AD patients who developed ACLF within 30 days after admission, the ROC curves associated to BE, DTE, CLIF-C-AD score, linear combination comprising H and T, in particular H and TG, marker linear combination according to formula 1 ("combination 3") and marker linear combination according to formula 2 ("combination 4") are shown in Fig. 11A and Fig. 11B. In Fig. 11C, the respective AUROC values are given as well as p-values.

The AUROC related to CLIF-C-AD score is 0.73 (0.67 - 0.79). The AUROC related to linear combination according to formula 1 (comprising H and T) is 0.76 (0.7 - 0.81). The CLIF-CAD score and the linear combination according to formula 1 show a similar performance. The AUROC related to marker linear combination according to formula 2 is 0.81 (0.76 - 0.86). Hence, the performance of marker linear combination according to formula 2 is improved in comparison to the CLIF-C-AD score as well as in comparison to the linear combination comprising H and T according to formula 1. This shows that the combination of a diagnostic score, such as the CLIF-C-AD score, and biophysical parameters determined from ESR spectra can improve the performance in comparison to common medical practice that considers the CLIF-C-AD score only. In particular, the combination of a diagnostic score, such as the CLIF-C-AD score, and biophysical parameters determined from ESR spectra can improve the performance to predict the development of AD, in comparison to common medical practice that considers the CLIF-C-AD score only.

In Fig. 12, the Kaplan-Meier survival analysis is shown. It shows that the binding efficiency has prognostic potential in predicting survival in AD patients and/or in ACLF patients.

Fig. 13 is related to the classification of samples to be associated to AD or ACLF. Fig. 13 shows ROC curves related to H, in particular H1A, C5, in particular C5B, and related to a linear combination comprising H (particularly H1A)

and KB, in particular KBB and KBC. Further ROC curves related to a linear combination comprising H (particularly H1A) and KB, in particular KBB and KBC, and S, in particular S1B is shown as well as ROC related to the marker linear combination related to formula 4 ("ACLF gg AD n"). The respective characteristic values are provided in Table 3.

Table 3: AUROC values, 95% confidence interval (95% CI), p values, sensitivities (sensi.) as well as specificities (speci.) are given. KH: linear combination comprising H1A, KBB and KBC; KHS: linear combination comprising H1A, KBB, KBC and S1B; combination formula 4: marker linear combination related to formula 4.

| | **AUROC** | **95% CI** | p | sensi. | speci. |
|---|---|---|---|---|---|
| H1A | 0,657 | 0,602 to 0,709 | | 80% | 34% |
| C5B | 0,637 | 0,582 to 0,690 | 0,69 | 80% | 36% |
| KH | 0,694 | 0,641 to 0,744 | 0,29 | 80% | 38% |
| KHS | 0,706 | 0,653 to 0,755 | 0,14 | 80% | 49% |
| Combination formula 4 | 0,723 | 0,671 to 0,772 | 0,07 | 80% | 46% |

Previous analysis shows that H1A is the biophysical parameter having the highest AUROC. This implies that with respect to a single biophysical parameter, H1A provides the best performance to discriminate between AD samples and ACLF samples. Exemplarily it is shown that the AUROC related to C5B is lower than the AUROC related to H1A.

A combination of at least two biophysical parameters can increase the AUROC. This means that a combination of biophysical parameters can improve the performance.

The data illustrates that the performance of combination KH is improved in comparison to H1A and C5B alone. The AUROC related to KH is greater than the AUROC related to H1A. The AUROC related to KH is also greater than the AUROC related to C5B.

The data further shows that the combination KHS shows an improvement of the performance in comparison to H1A, C5B or combination KH, in classifying a sample to be associated to ACLF or to be associated to AD.

In comparison to the AUROC related to H1A, the AUROC associated to the marker linear combination related to formula 4 is increased. The AUROC associated to the marker linear combination related to formula 4 is also increased in comparison to the AUROC related to C5B, combination KH, and combination KHS. The significance in the case of marker linear combination related to formula 4 is higher than the significance related to KHS (KHS: $p = 0.14$; marker linear combination related to formula 4: $p = 0.07$).

The data shows that at sensitivity of 80%, the specificity of marker linear combination related to formula 4 is greater than specificity related to H1A to classifying a sample to be associated to ACLF or to be associated to AD. In particular, the specificity is increased by 12%. This means that at sensitivity of 80%, 12% more patients get the correct diagnosis. This means 12% more patients are classified correctly. Hence, Fig. 13 and the associated Table 3 illustrate the improvement of the performance when considering marker linear combination related to formula 4, instead of H1A, C5B or combination KH for the classification of a sample to be associated to ACLF or to be associated to AD.

Taken together, a combination comprising the hydrophobicity, in particular hydrophobicity of high-affinity binding site, the binding constant and the order parameter can show an improved performance to classify AD and ACLF patients correctly. In particular, the marker linear combination according to formula 4 can be used to discriminate between AD and ACLF patients.

Fig. 14 is associated to the classification of CC and AD samples. Fig. 14A and Fig. 14B show ROC curves related to alpha, C5B, a combination comprising alpha and C5, in particular C5B, and related to a linear combination comprising alpha and C5, particularly C5B, and C3, particularly C3A and C3B. Further, ROC curves related to the marker linear combination related to formula 3 ("AD_vs_CC") are shown. The respective characteristic values are provided in Table 4. Table 4 gives additional information about ROC curves associated to C3A, C3B, BE, and a combination of alpha and C3, in particular alpha, C3A and C3B.

Table 4: AUROC values, 95% confidence interval (95% CI), p values, sensitivities (sensi.) as well as specificities (speci.) are given. AC5: linear combination comprising alpha and C5B; AC3: linear combination comprising alpha, C3A and C3B; AC5C3: linear combination comprising alpha, C5B, C3A and C3B; combination formula 3: marker linear combination related to formula 3.

| | **AUROC** | **95% CI** | p | sensi. | speci. |
|---|---|---|---|---|---|
| Alpha | 0,865 | 0,809 to 0,910 | | 72% | 83% |

(continued)

| | AUROC | 95% CI | p | sensi. | speci. |
|---|---|---|---|---|---|
| C3B | 0,856 | 0,799 to 0,902 | 0,40 | 75% | 83% |
| C5B | 0,863 | 0,806 to 0,908 | 0,94 | 89% | 83% |
| BE | 0,853 | 0,796 to 0,900 | 0,04 | 68% | 83% |
| C3A | 0,853 | 0,795 to 0,899 | 0,03 | 68% | 83% |
| BE_DTE | 0,862 | 0,805 to 0,907 | 0,83 | 76% | 83% |
| BE_C5B | 0,876 | 0,821 to 0,919 | 0,71 | 88% | 83% |
| C3B_C5B | 0,880 | 0,826 to 0,922 | 0,62 | 89% | 83% |
| AC5 | 0,893 | 0,841 to 0,933 | 0,24 | 89% | 83% |
| AC3 | 0,870 | 0,815 to 0,914 | 0,56 | 72% | 83% |
| AC3C5 | 0,925 | 0,878 to 0,958 | 0,02 | 88% | 83% |
| Combination formula 3 | 0,923 | 0,877 to 0,957 | 0,03 | 91% | 83% |

Previous analysis shows that alpha is the biophysical parameter having the highest AUROC. This implies that with respect to a single biophysical parameter, alpha provides the best performance to discriminate between AD samples and CC samples. Exemplarily it is shown that the AUROC related to C5B, C3A, C3B or BE is lower than the AUROC related to alpha.

A combination of at least two biophysical parameters can increase the AUROC. This means that a combination of biophysical parameters can improve the performance. For instance, the AUROC related to combination AC5 (0.893 (0.841 - 0.933)) is greater than the AUROC related to a single biophysical parameter such as C3A, BE, C5B, C3B or alpha. The AUROC related to combination AC3 (0.87 (0.815 - 0.914)) is also greater than the AUROC related to a single biophysical parameter such as C3A, BE, C5B, C3B or alpha.

The data further shows that not every combination of at least two biophysical parameters increases the AUROC. This means that not every combination of at least two parameters can provide an increased performance to classify samples to a particular disease, in particular to an increased performance to classify samples to be associated to AD or CC. Exemplarily, the AUROC of the combination of BE (binding efficiency) and DTE (detoxification efficiency) is given (BE_DTE). It is lower than the AUROC of alpha alone. Hence, the performance of the combination BE_DTE is worse than the performance of alpha (to classify samples to be associated to AD or CC). The combination comprising one of a spectral component from free spin probe molecules (C3), a spectral component from spin probe on lipid-fraction of serum (C5), or a geometry factor (alpha) increases the performance. In particular, the AUROC of the combination BE_C5B is greater than the AUROC of alpha. The AUROC of the combination BE_C5B is also greater than the AUROC of the combination BE_DTE. A combination comprising a plurality of a spectral component from free spin probe molecules (C3), a spectral component from spin probe on lipid-fraction of serum (C5), or a geometry factor (alpha) further increase the AUROC. This means that a combination comprising a plurality of a spectral component from free spin probe molecules (C3), a spectral component from spin probe on lipid-fraction of serum (C5), or a geometry factor (alpha) further increase the performance, in particular the performance to discriminate between a sample associated to AD and a sample associated to CC.

The linear combination comprising alpha, C3 and C5 increases partially significantly the AUROC (0.925 (0.878 - 0.958)) in comparison to the AUROC related to alpha (0.865 (0.809 - 0.91)). The marker linear combination according to formula 3 shows an AUROC of 0.923 (0.877 - 0.957) that is partially significantly greater than the AUROC related to alpha.

Data shows exemplarily, that at specificity of 83% the sensitivity of the marker linear combination according to formula 3 is 91% and, hence, 19% larger than the sensitivity related to alpha alone. The marker linear combination according to formula 3 can improve the classification of samples to be related to CC or AD.

The ROC curves shown in Fig. 14B, further illustrate that the ROC curve related to C5B is serrated. In contrast, the ROC curve related to combination AC5C3 is curved. The curved ROC curve is much more robust than the serrated ROC curve.

Fig. 15 and associated Table 5 show ROC curves and data to discriminate between CC and AD patients in case the AD is in some patients accompanied by a bacterial infection.

Table 5: AUROC values, 95% confidence interval (95% CI), p values, sensitivities (sensi.) as well as specificities (speci.) are given. AC5: linear combination comprising alpha and C5B; AC5C3: linear combination comprising alpha, C5B, C3A and C3B; combination formula 3: marker linear combination related to formula 3.

| | **AUROC** | **95% CI** | P | sensi. | speci. |
|---|---|---|---|---|---|
| Alpha | 0,882 | 0,836 to 0,918 | | 75% | 83% |
| AC5 | 0,907 | 0,865 to 0,939 | 0,24 | 91% | 83% |
| AC5C3 | 0,938 | 0,902 to 0,964 | 0,02 | 90% | 83% |
| Combination formula 3 | 0,936 | 0,900 to 0,963 | 0,02 | 93% | 83% |

Also in case of a bacterial infection, a combination of biophysical parameters can improve the performance (to discriminate between AD and CC) in comparison to the performance of a single parameter, such as alpha. The combination AC5C3 as well as the marker linear combination according to formula 3 strongly increase the AUROC.

At a specificity of 83%, the sensitivity according to marker linear combination according to formula 3 (93%) is considerably increased in comparison to the sensitivity of alpha only (75%). Further, the sensitivity of combination AC5 (91%) as well as sensitivity of combination AC5C3 (90%) are increased compared to the sensitivity of alpha.

Hence, the marker linear combination according to formula 3 can be used to discriminate between AD and CC patients in case of a bacterial infection as well as in absence of an accompanied bacterial infection.

According to an embodiment of the method, it can be used to estimate the development of a disease, in particular to predict the development of AD, in particular to predict the development of AD to ACLF.

In Fig. 16, the ROC curves related to T, in particular TG, in particular TG2A, CLIF-C-AD score, a combination comprising H (particularly H1B) and T (particularly TG2A) are shown. Further, the ROC curve related to the marker linear combination according to formula 2 is shown. In Table 6, the associated characteristics are provided.

Table 6: AUROC values, 95% confidence interval (95% CI), p values, sensitivities (sensi.) as well as specificities (speci.) are given. HT: linear combination comprising H1B and TG2A; combination formula 2: marker linear combination related to formula 2.

| | **AUROC** | **95% CI** | p | sensi. | speci. |
|---|---|---|---|---|---|
| TG2A | 0,733 | 0,673 to 0,788 | | 81% | 57% |
| CLIF-C-AD | 0,733 | 0,672 to 0,787 | 0,99 | 81% | 55% |
| HT | 0,761 | 0,703 to 0,814 | 0,34 | 81% | 60% |
| Combination formula 2 | 0,813 | 0,758 to 0,860 | 0,01 | 81% | 76% |

The performance of biophysical parameter TG2A is as good as the performance of the CLIF-C-AD score, to particularly predict the development of AD. Both show identical AUROCs and comparable specificity at a sensitivity of 81%. A combination of two appropriate biophysical parameters can improve the performance. In particular, the combination HT shows a greater AUROC (0.761 (0.703 - 0.814) in comparison to TG2A (0.733 (0.673 - 0.788)) as well as in comparison to CLIF-C-AD score (0.733 (0.672 - 0.787)). Hence, the performance, in particular the performance to predict the development of AD, of the combination HT is improved in comparison to the CLIF-C-AD score that is used for diagnosis and prognosis in clinical practice.

The marker linear combination according to formula 2 further increases the AUROC (0.813 (0.758 - 0.86)). Compared to the AUROC of TG2A, the increase is significant.

At a sensitivity of 81%, the specificity of TG2A is 57%. At sensitivity of 81%, the specificity according to the CLIF-C-AD score is 55%. The marker linear combination according to formula 2 shows a specificity of 76% (at sensitivity 81%). Hence, in comparison to TG2A, the specificity increases by 19% when considering the marker linear combination according to formula 2. In comparison to the CLIF-C-AD score, the specificity increases by 21% when considering the marker linear combination according to formula 2.

In particular, the marker linear combination according to formula 2 can be used to predict the development of AD.

## Claims

1. A method performed in a computing device, the method comprising the steps of:

   receiving at the computing device a plurality of spectra acquired from a corresponding plurality of aliquots containing a biophysiological carrier protein, wherein at least one of a concentration of a spin probe and a concentration of a polar reagent varies between the aliquots;
   determining by the computing device biophysical parameters based on the received spectra;
   applying by the computing device the biophysical parameters as an input to a marker linear combination, wherein the marker linear combination comprises a predefined combination of at least two biophysical parameters,
   the computing device using the marker linear combination to determine the input parameters relating to one or more of said predetermined diseases, and
   outputting a result according to the marker linear combination,

   **characterised in that** the marker linear combination comprises at least one of the biophysical parameters:

   - a binding constant of a spin probe (KB),
   - a polarity surrounding a spin probe (H),
   - an order parameter of a spin probe (S),
   - a rotation correlation time of a spin probe (T),
   - a spectral component from free spin probe molecules (C3),
   - a spectral component from spin probe on lipid-fraction of serum (C5), or
   - a geometry factor (alpha).

2. Method according to claim 1, **characterised in that** the marker linear combination comprises at least one of the biophysical parameters:

   - a binding constant of a spin probe (KB),
   - a polarity surrounding a spin probe (H),
   - an order parameter of a spin probe (S),
   - a rotation correlation time of a spin probe (T),
   - a spectral component from free spin probe molecules (C3), or
   - a spectral component from spin probe on lipid-fraction of serum (C5).

3. Method according to one of the claims 1 or 2, in that the marker linear combination comprises at least one of the biophysical parameters:

   - a spectral component from free spin probe molecules (C3),
   - a spectral component from spin probe on lipid-fraction of serum (C5),
   - a rotation correlation time of a spin probe (T),
   - an order parameter of a spin probe (S),
   or
   - a polarity surrounding a spin probe (H),
   in particular
   - a spectral component from free spin probe molecules (C3),
   - a spectral component from spin probe on lipid-fraction of serum (C5),
   or
   - a polarity surrounding a spin probe (H).

4. Method according to one of the claims 1 to 3, **characterised in that** the marker linear combination comprises at least one of:

   - a combination of a spectral component from spin probe on lipid-fraction of serum (C5) and a spectral component from free spin probe molecules (C3),
   - a combination of a spectral component from spin probe on lipid-fraction of serum (C5) and a geometry factor (alpha),
   - a combination of a spectral component from free spin probe molecules (C3) and a geometry factor (alpha),
   - an order parameter of a spin probe (S),

or
- a polarity surrounding a spin probe (H).

**5.** Method according to one of the claims 1 to 4, **characterised in that** the marker linear combination comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha),
- a combination of a spectral component from free spin probe molecules (C3) and a geometry factor (alpha),
- a combination of a polarity surrounding a spin probe (H) and a rotation correlation time of a spin probe (T),
- a combination of a polarity surrounding a spin probe (H) and an order parameter of a spin probe (S),
- a combination of a polarity surrounding a spin probe (H) and a binding constant of a spin probe (KB), or
- a combination of an order parameter of a spin probe (S) and a binding constant of a spin probe (KB).

**6.** Method according to one of the claims 1 to 5, **characterised in that** the marker linear combination comprises:

- a combination of a polarity surrounding a spin probe (H) and a rotation correlation time of a spin probe (T), in particular a combination of a polarity surrounding a spin probe, in particular a polarity surrounding a first spin probe in the solution B (H1B) and a rotation correlation time of a spin probe (T), in particular a rotation correlation time of a second spin probe in the solution A (TG2A).

**7.** Method according to claim 6, **characterised in that** the marker linear combination comprises:

- a combination of a polarity surrounding a spin probe (H) and a rotation correlation time of a spin probe (T), and
- a score, in particular a CLIF-C-AD-Score,

in particular a combination of a polarity surrounding a spin probe, in particular a polarity surrounding a first spin probe in the solution B (H1B) and a rotation correlation time of a spin probe, in particular a rotation correlation time of a second spin probe in the solution A (TG2A) and the CLIF-C-AD score.

**8.** Method according to one of the claims 1 to 5, **characterised in that** the marker linear combination comprises at least one of:

- a combination of a spectral component from spin probe on lipid-fraction of serum (C5) and a spectral component from free spin probe molecules (C3),
- a combination of a spectral component from spin probe on lipid-fraction of serum (C5) and a geometry factor (alpha), or
- a combination of a spectral component from free spin probe molecules (C3) and a geometry factor (alpha).

**9.** Method according to claim 8, **characterised in that** the marker linear combination comprises:
a combination of a spectral component from spin probe on lipid-fraction of serum (C5), a spectral component from free spin probe molecules (C3), and a geometry factor (alpha) or a combination of a spectral component from spin probe on lipid-fraction of serum (C5) a spectral component from free spin probe molecules (C3), a geometry factor (alpha) and a binding efficiency of a spin probe (BE).

**10.** Method according to one of the claims 8 or 9, **characterised in that** the marker linear combination comprises:

- a combination of a binding efficiency of a spin probe (BE), a geometry factor (alpha), a spectral component from free spin probe molecules, in particular a spectral component from free spin probe molecules in the solution A (C3A) and a spectral component from free spin probe molecules in the solution B (C3B), and a spectral component from spin probe on lipid-fraction of serum, in particular a spectral component from spin probe on lipid-fraction of serum in the solution B (C5B).

**11.** Method according to one of the claims 1 to 5, **characterised in that** the marker linear combination comprises at least one of:

- a combination of a polarity surrounding a spin probe (H) and an order parameter of a spin probe (S),
- a combination of a polarity surrounding a spin probe (H) and a binding constant of a spin probe (KB), or
- a combination of a order parameter of a spin probe (S) and a binding constant of a spin probe (KB).

**12.** Method according to claim 11, **characterised in that** the marker linear combination comprises:

- a combination of a polarity surrounding a spin probe (H) and an order parameter of a spin probe (S), and a binding constant of a spin probe (KB).

**13.** Method according to one of the claims 11 or 12, **characterised in that** the marker linear combination comprises:

- a combination of a polarity surrounding a spin probe (H), an order parameter of a spin probe (S) and a binding constant of a spin probe (KB),

and at least one of:

- a binding efficiency of a spin probe (BE),
- a detoxification efficiency (DTE),
- a spectral component from spin probe on lipid-fraction of serum (C5), or
- a rotation correlation time of a spin probe (T).

**14.** Method according to one of the claims 11 to 13, **characterised in that** the marker linear combination comprises:

- a combination of a binding efficiency of a spin probe (BE), a detoxification efficiency (DTE), a binding constant of a spin probe, in particular of a binding constant of a spin probe in a solution B (KBB) and a binding constant of a spin probe in a solution C (KBC), a spectral component from spin probe on lipid-fraction of serum, in particular a spectral component from spin probe on lipid-fraction of serum in the solution B (C5B), a polarity surrounding a spin probe, in particular a polarity surrounding a first spin probe in a solution A (H1A), an order parameter of a spin probe, in particular an order parameter of the first spin probe in the solution B (S1B), and a rotation correlation time of a spin probe, in particular a rotation correlation time of a second spin probe in the solution A (TG2A).

**15.** Method according to one of the claims 1 to 14, **characterised in that** said predetermined diseases is a hepatic disease, a stage of a hepatic disease and/or a predefined hepatic function, in particular an abnormal hepatic function, in particular one of: a compensated cirrhosis, a cirrhosis with acute decompensation, an acute on chronic liver failure, an insensitivity with respect to a therapy, a prognostic information regarding a risk of death in a certain time frame or a risk of deterioration of a disease.

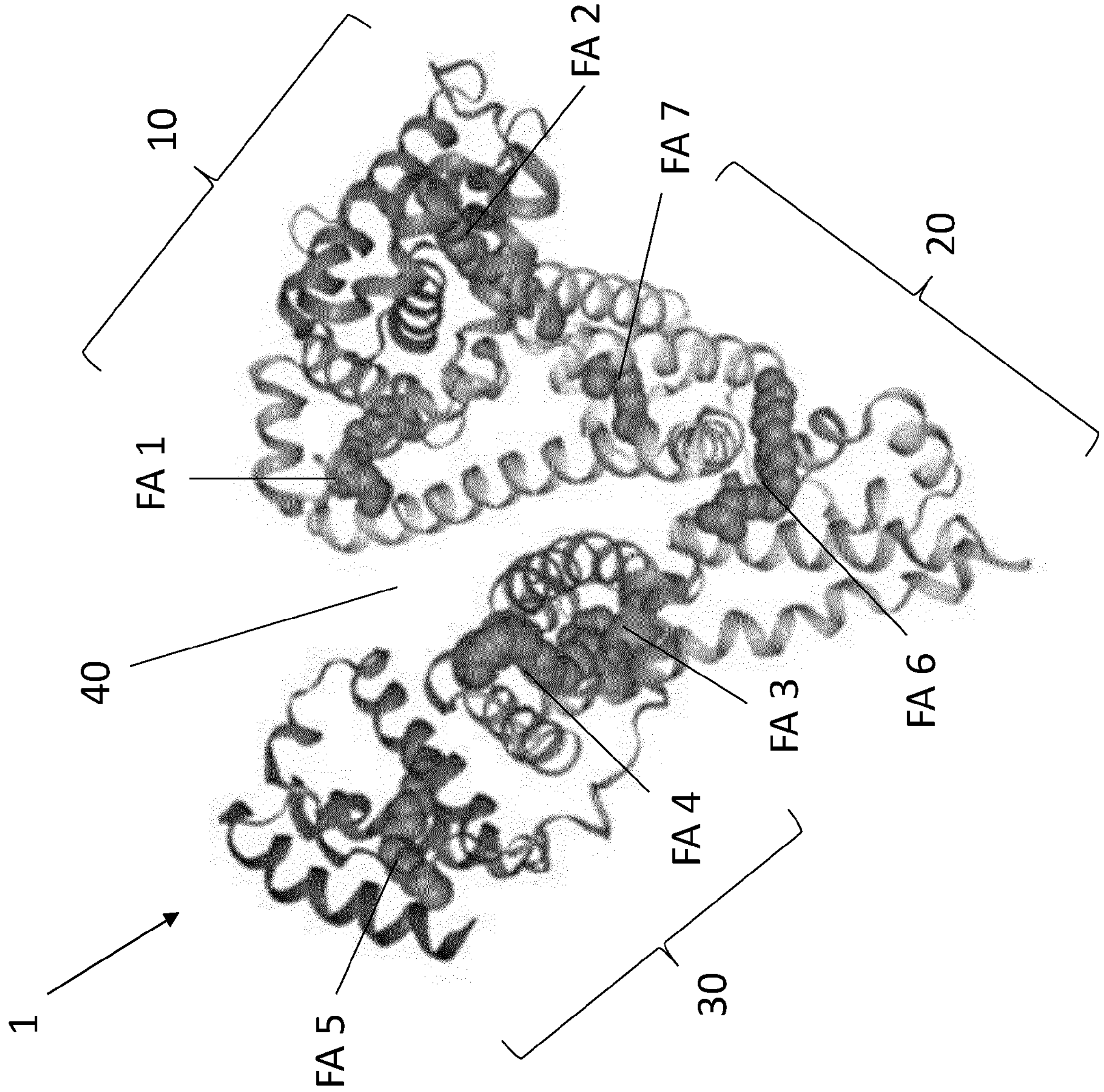


Fig. 1

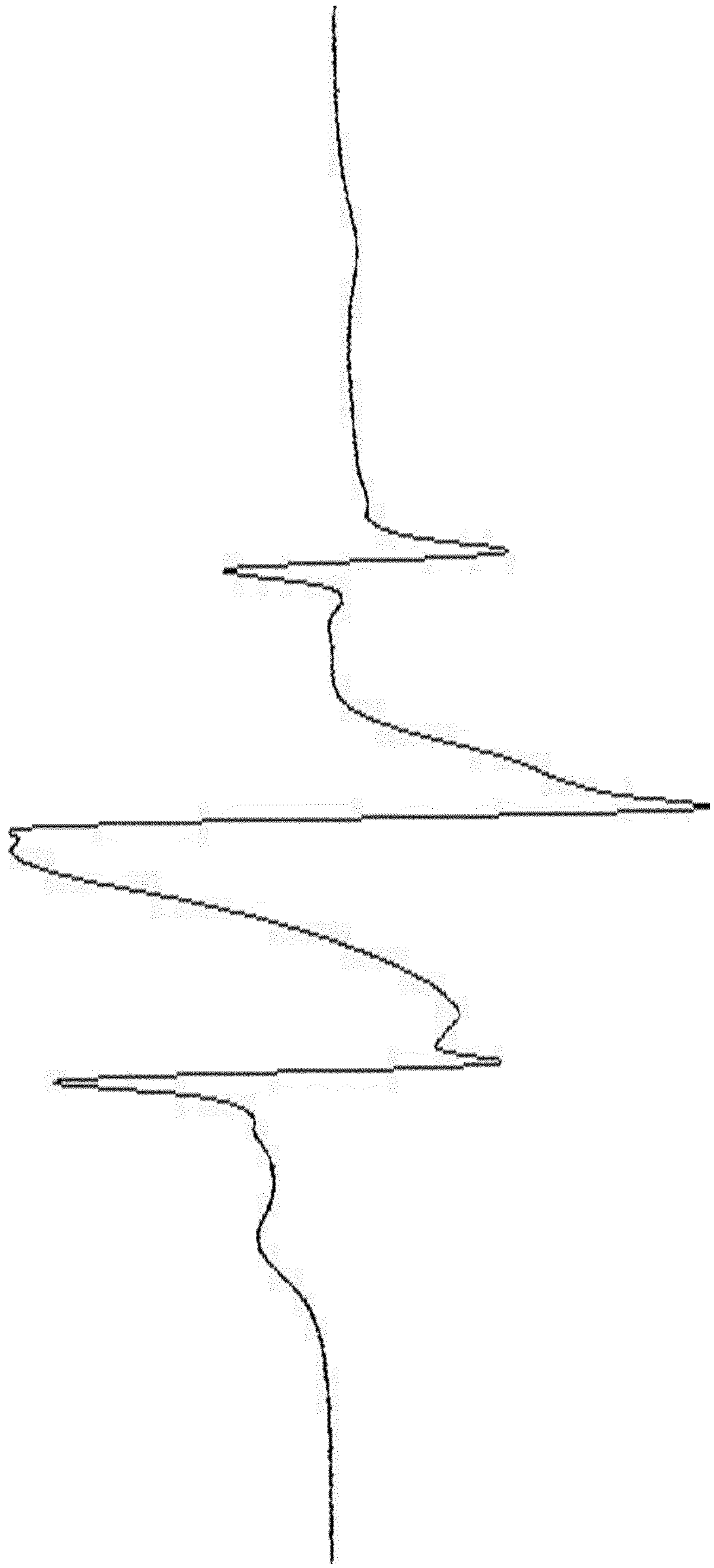

Fig. 2

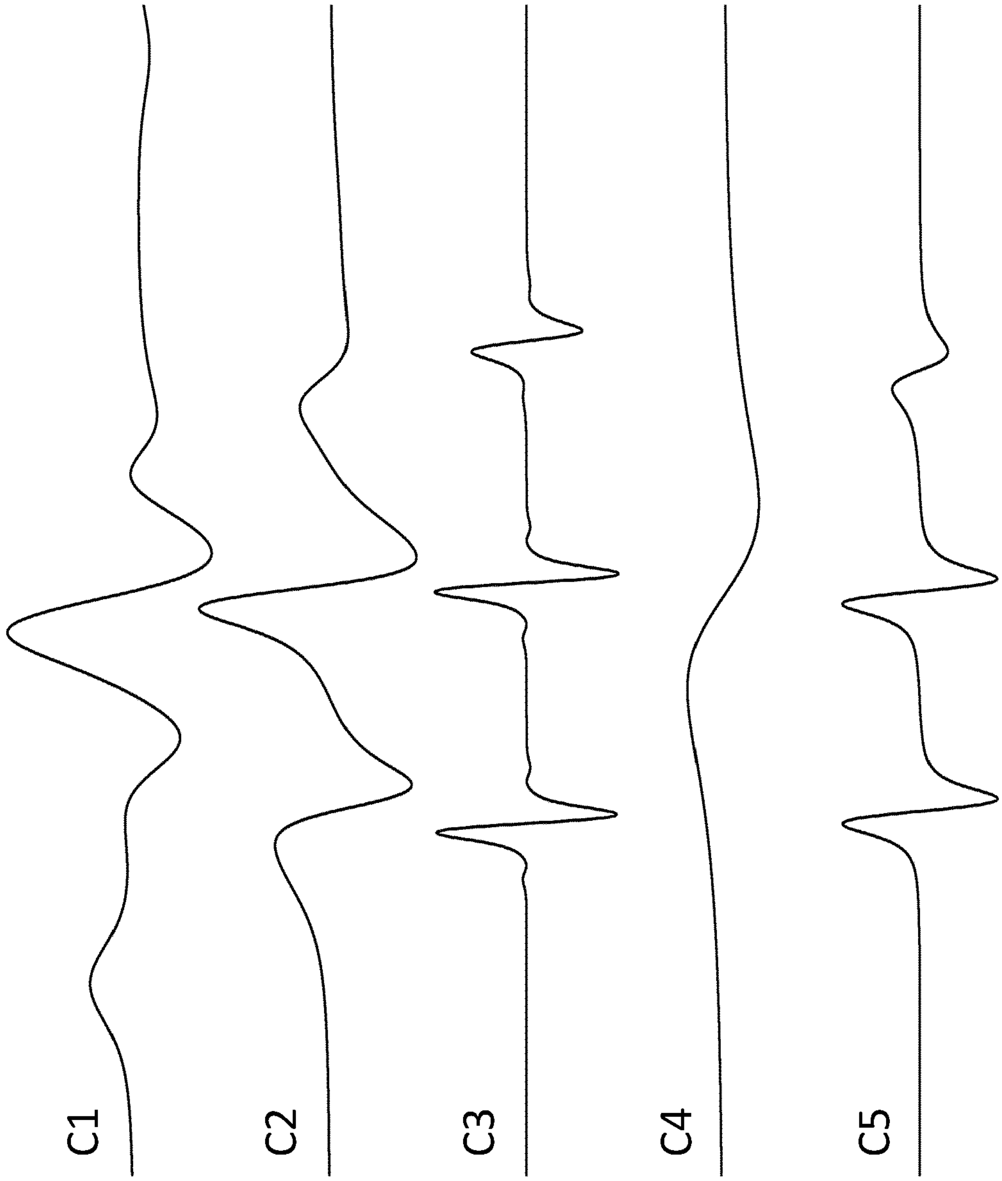


Fig. 3

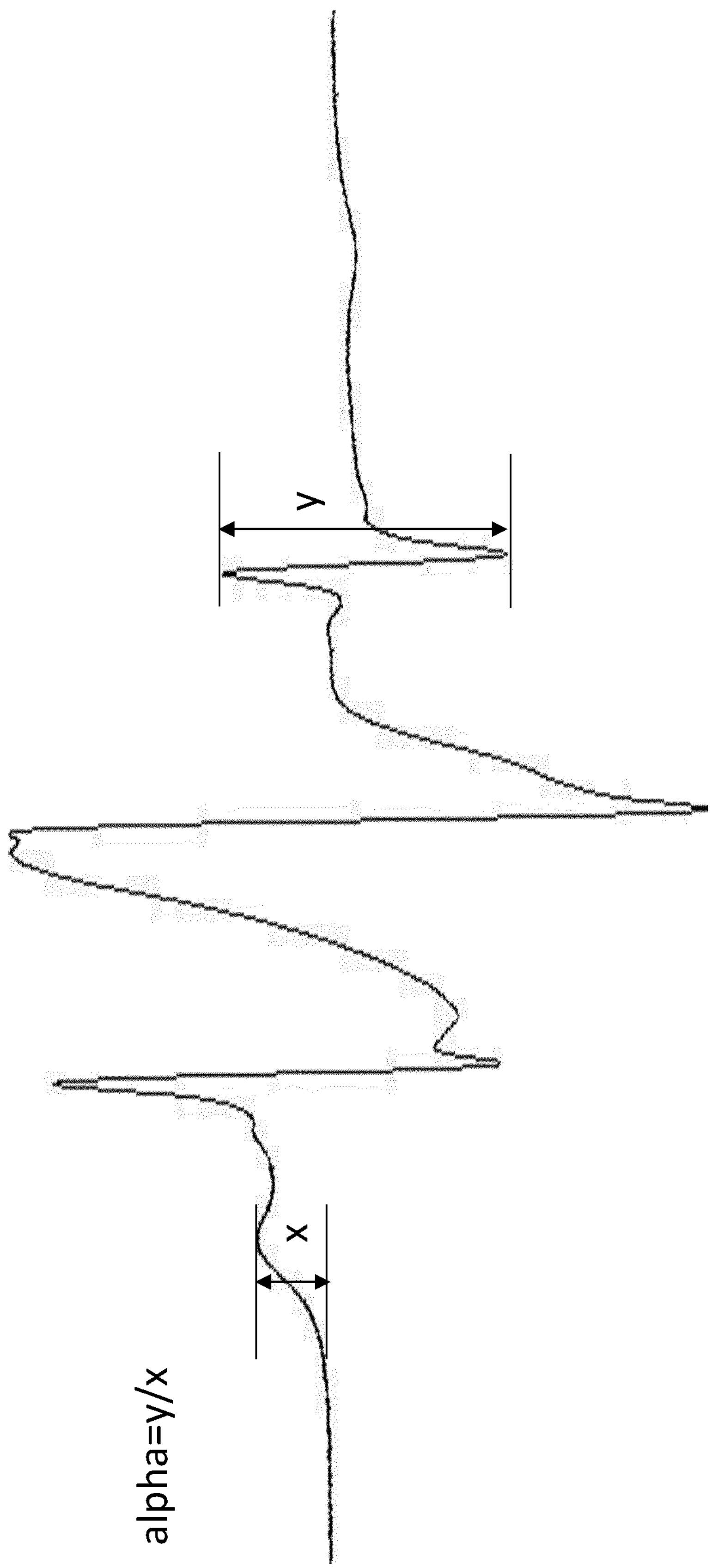


Fig. 4

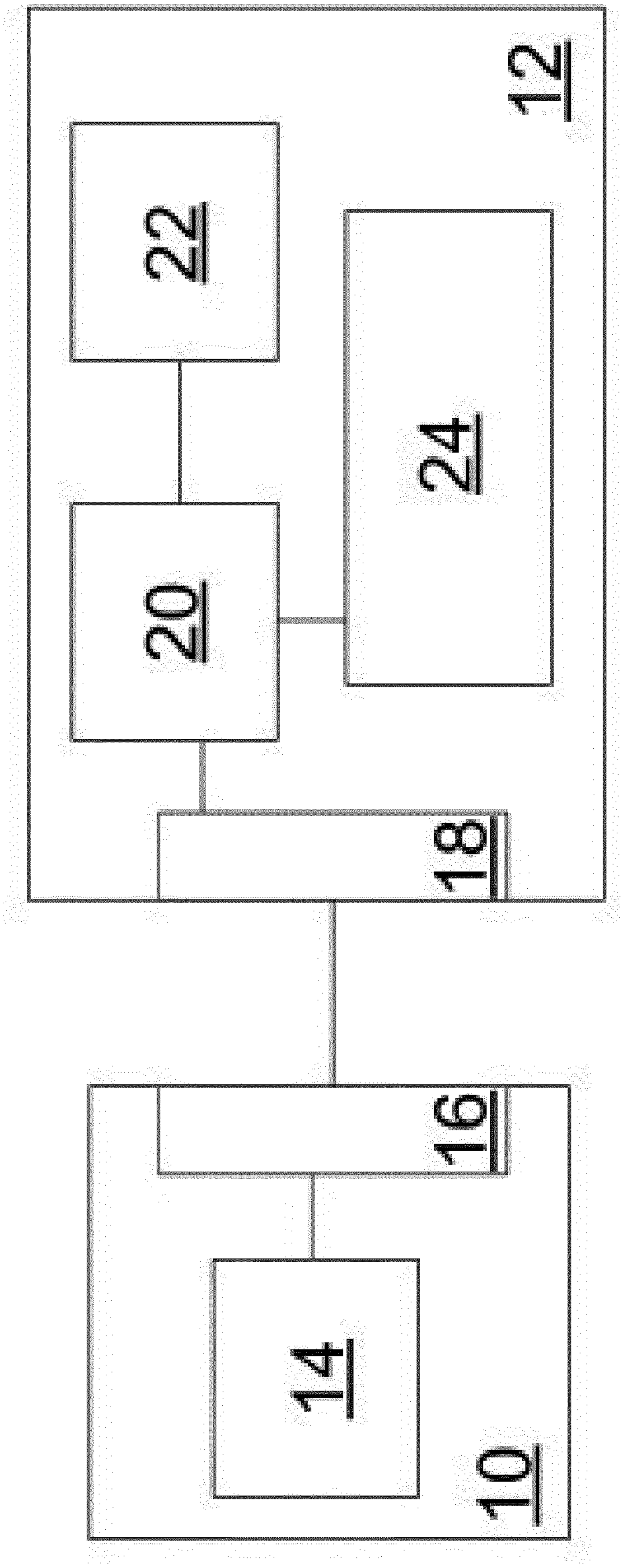


Fig. 5

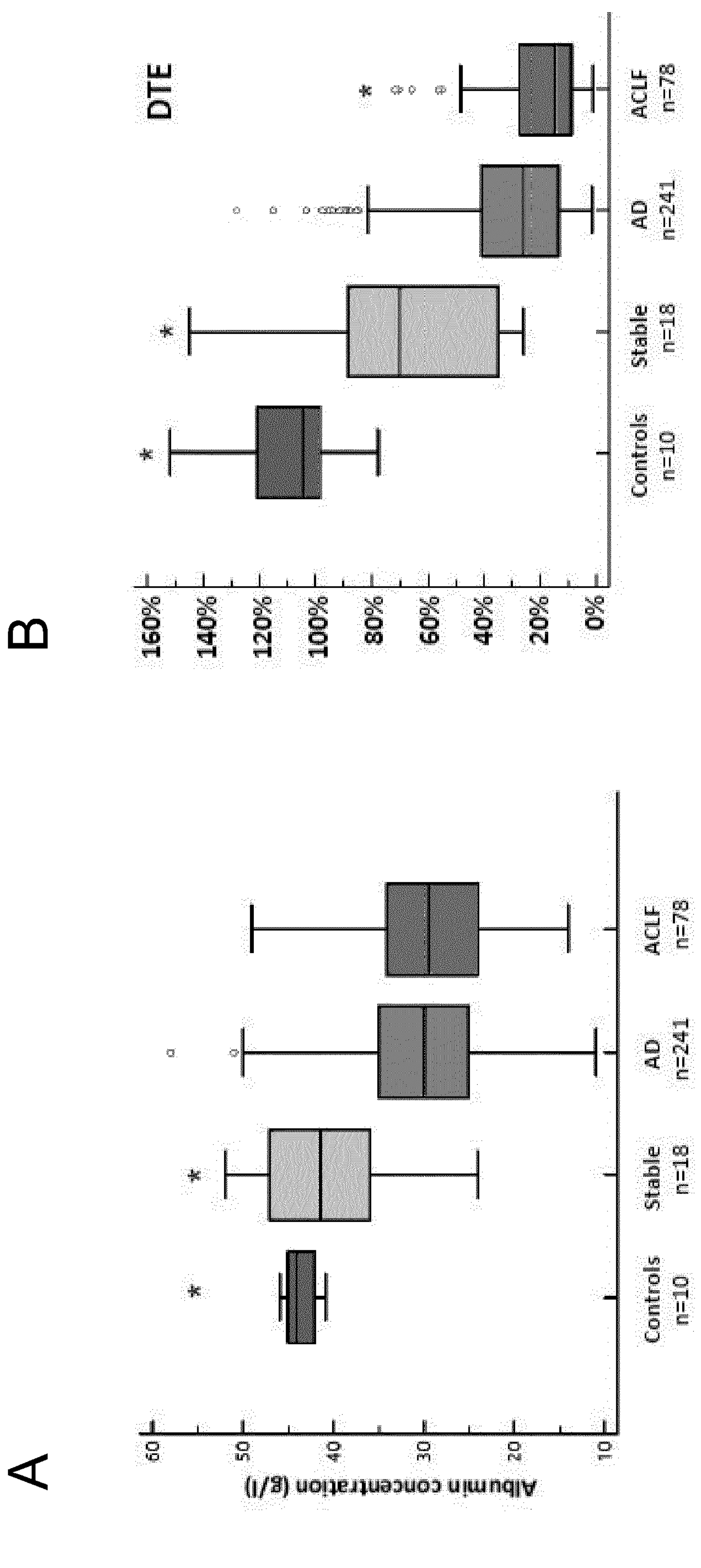
A
Albumin concentration (g/l)
60
50
40
30
20
10
Controls n=10
Stable n=18
AD n=241
ACLF n=78
B
DTE
160%
140%
120%
100%
80%
60%
40%
20%
0%
Controls n=10
Stable n=18
AD n=241
ACLF n=78

Fig. 6

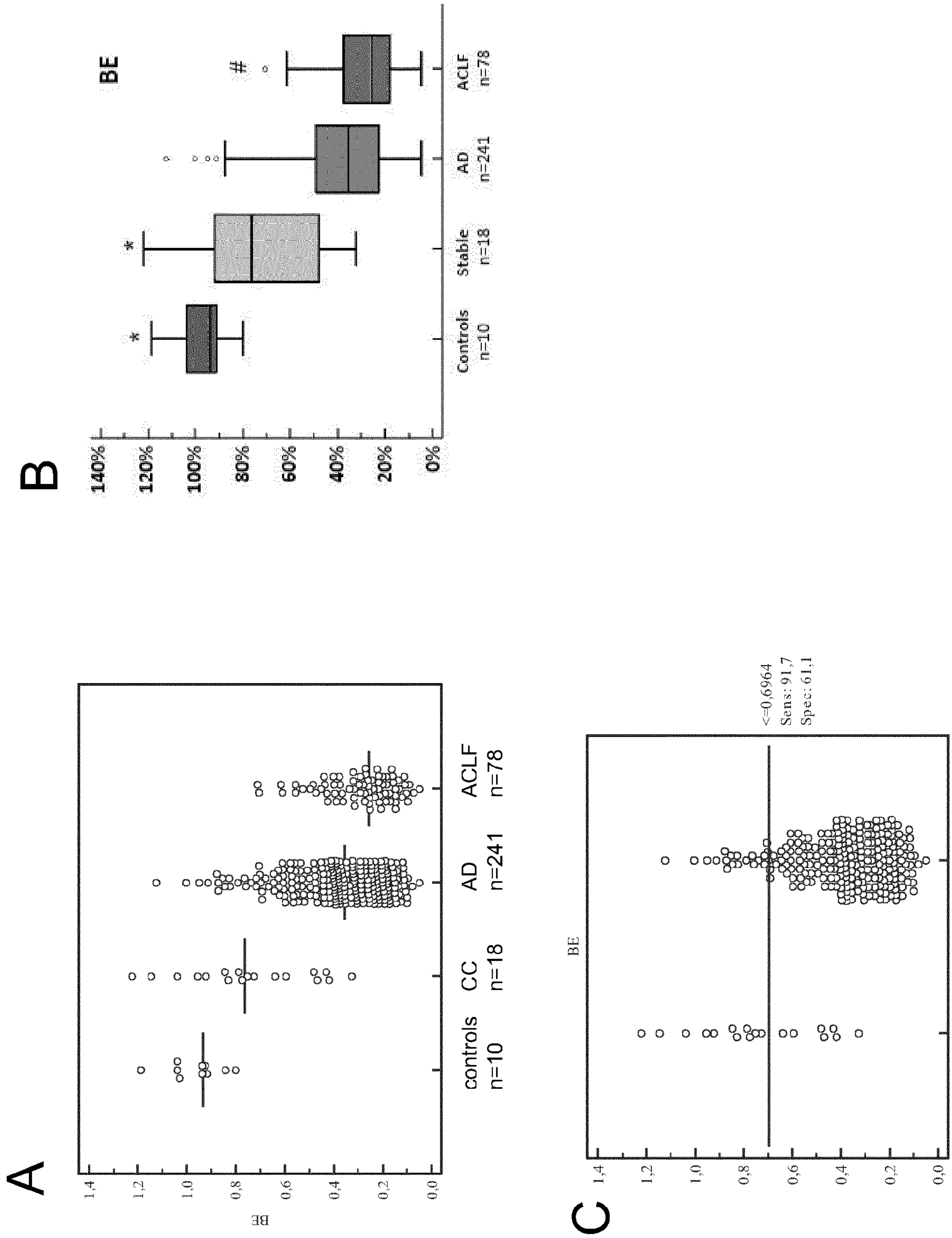
A
BE
1,4
1,2
1,0
0,8
0,6
0,4
0,2
0,0
controls n=10
CC n=18
AD n=241
ACLF n=78
B
BE
140%
120%
100%
80%
60%
40%
20%
0%
*
*
#
Controls n=10
Stable n=18
AD n=241
ACLF n=78
C
BE
<=0,6964
Sens: 91,7
Spec: 61,1
CC n=18
AD n=241

Fig. 7

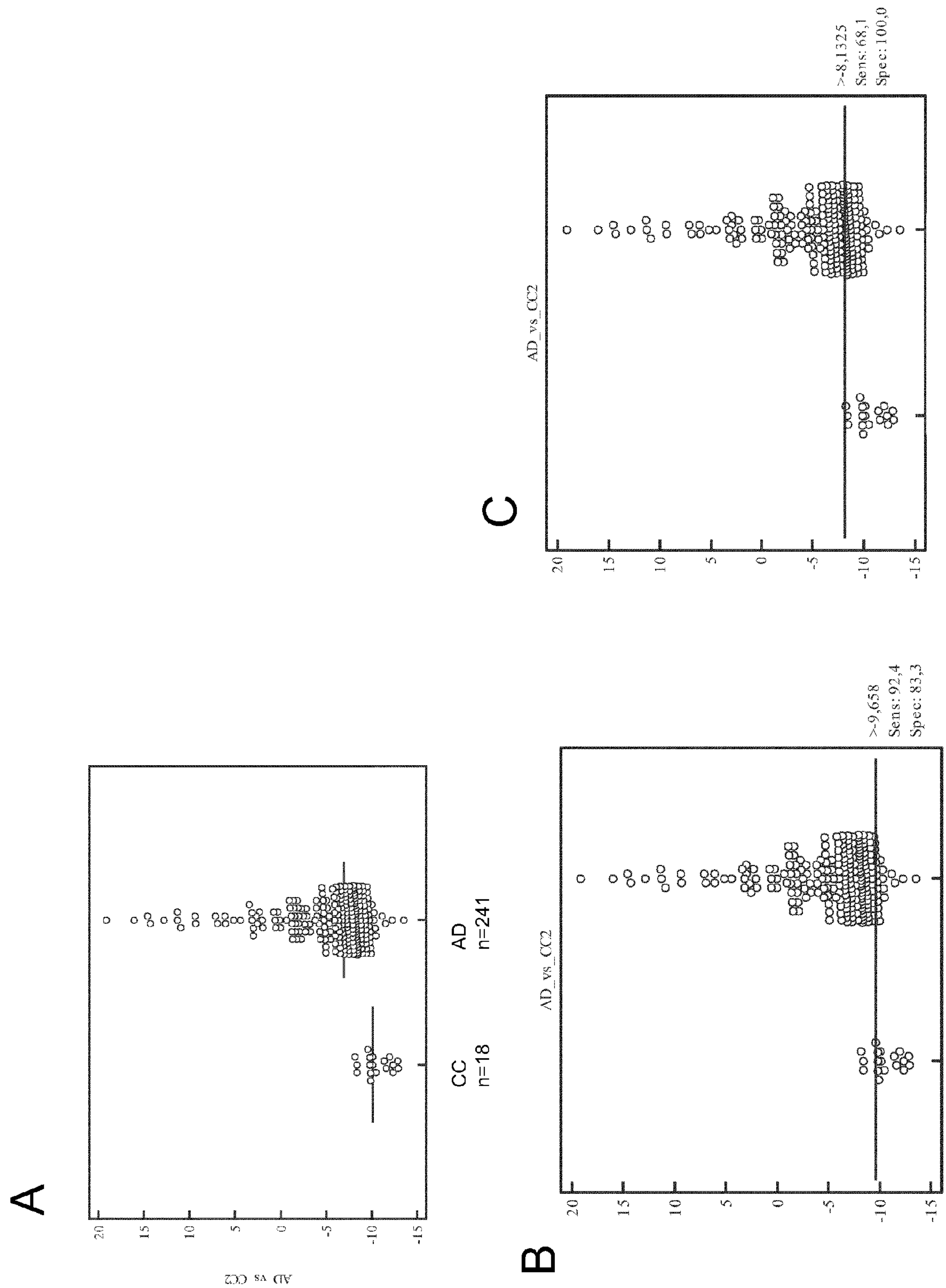
A
AD vs CC2
CC
n=18
AD
n=241
B
AD_vs_CC2
>-9,658
Sens: 92,4
Spec: 83,3
CC
n=18
AD
n=241
C
AD_vs_CC2
>-8,1325
Sens: 68,1
Spec: 100,0
CC
n=18
AD
n=241

Fig. 8

A

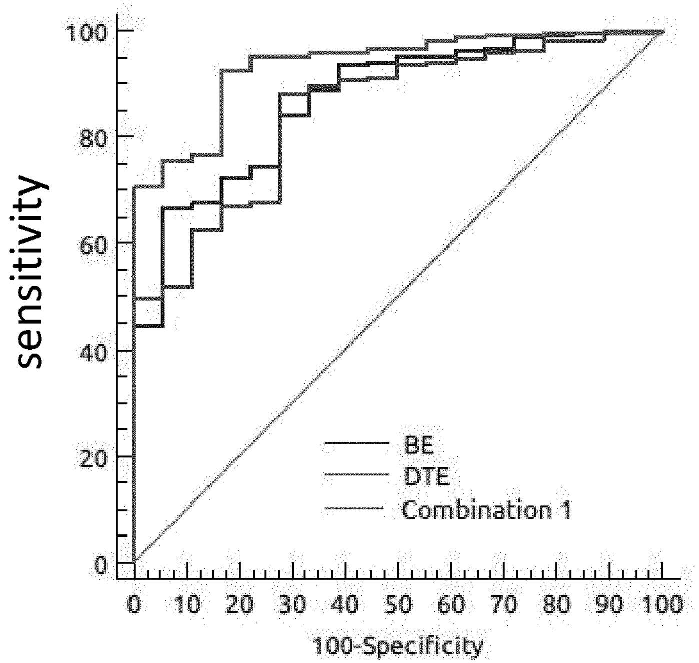


B

Comparison of AUROC:

| | |
|---|---|
| BE | 0.87 (0.82-0.91) |
| DTE | 0.85 (0.80-0.89) |
| Combination 1 | 0.94 (0.90-0.96) |

| | |
|---|---|
| BE~DTE | p=0.10 |
| BE~Combination 1 | p=0.01 |
| DTE~Combination 1 | p=0.01 |

Fig. 9

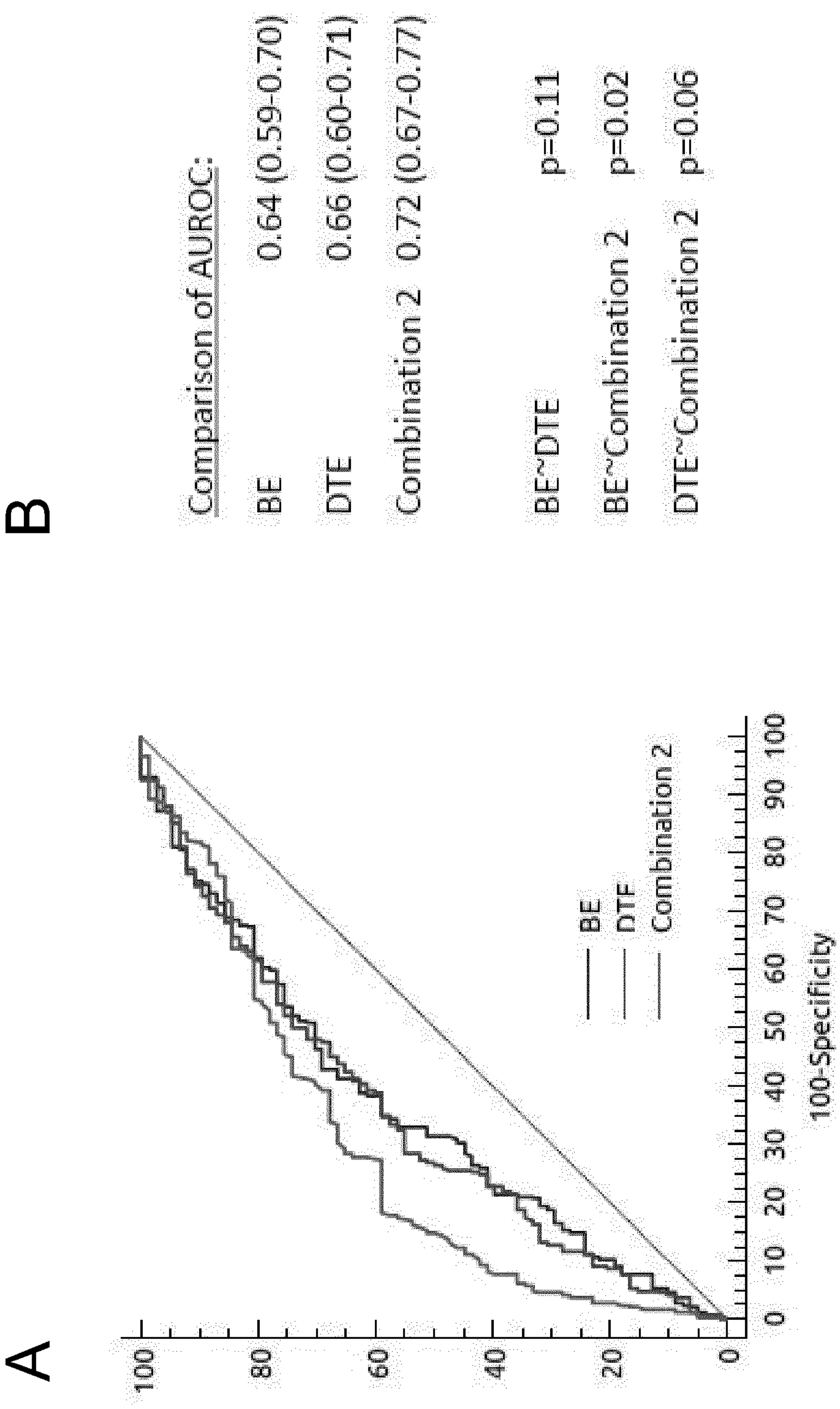
A
100
80
60
40
20
0
0 10 20 30 40 50 60 70 80 90 100
100-Specificity
BE
DTE
Combination 2
B
Comparison of AUROC:
BE 0.64 (0.59-0.70)
DTE 0.66 (0.60-0.71)
Combination 2 0.72 (0.67-0.77)
BE~DTE p=0.11
BE~Combination 2 p=0.02
DTE~Combination 2 p=0.06

Fig. 10

A

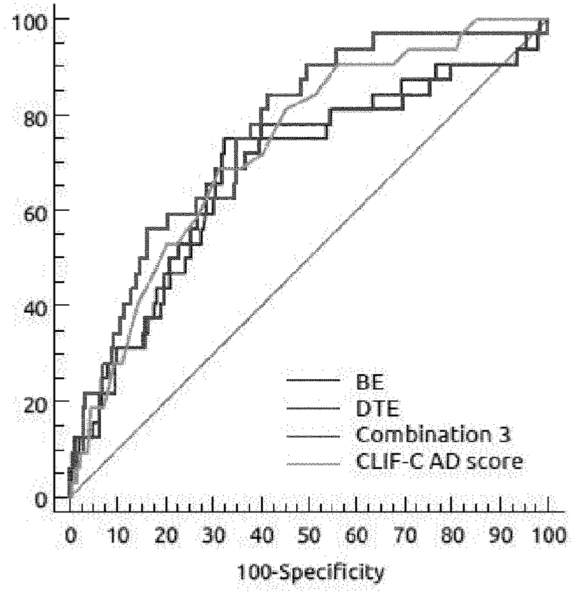


B

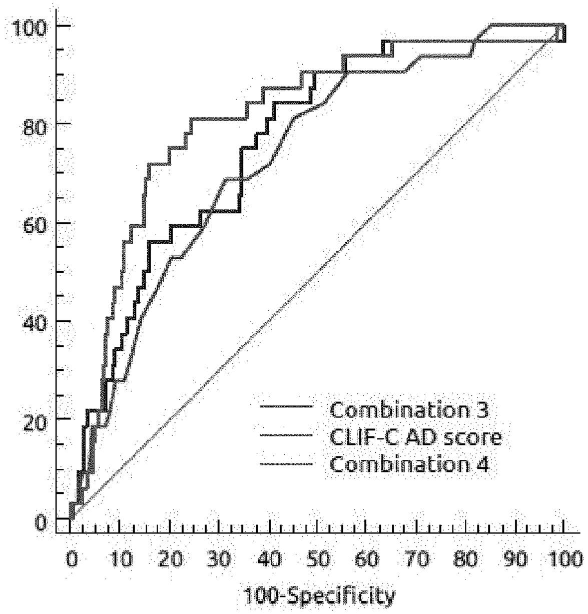


C

<u>Comparison of AUROC</u>:

| | |
|---|---|
| Combination 3 | 0.76 (0.70-0.81) |
| CLIF-C AD | 0.73 (0.67-0.79) |
| Combination 4 | 0.81 (0.76-0.86) |

| | |
|---|---|
| Comb.3~CLIF-C AD | p=0.60 |
| Comb.3~Comb.4 | p=0.07 |
| CLIF-C AD~Comb.4 | p=0.01 |

Fig. 11

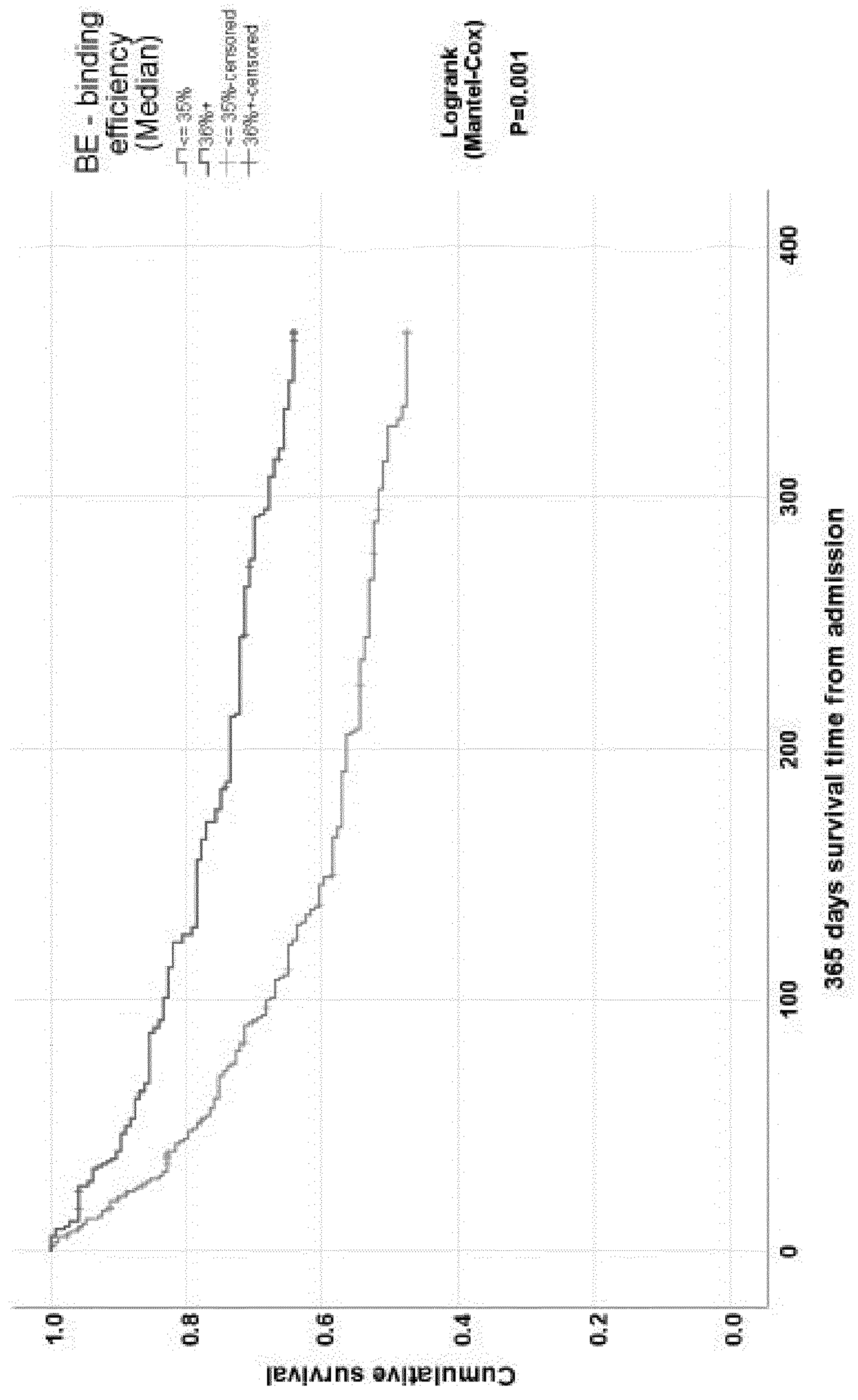
BE - binding efficiency (Median)
<= 35%
36%+
<= 35%-censored
36%+-censored
Logrank (Mantel-Cox)
P=0.001
Cumulative survival
1.0
0.8
0.6
0.4
0.2
0.0
0
100
200
300
400
365 days survival time from admission

Fig. 12

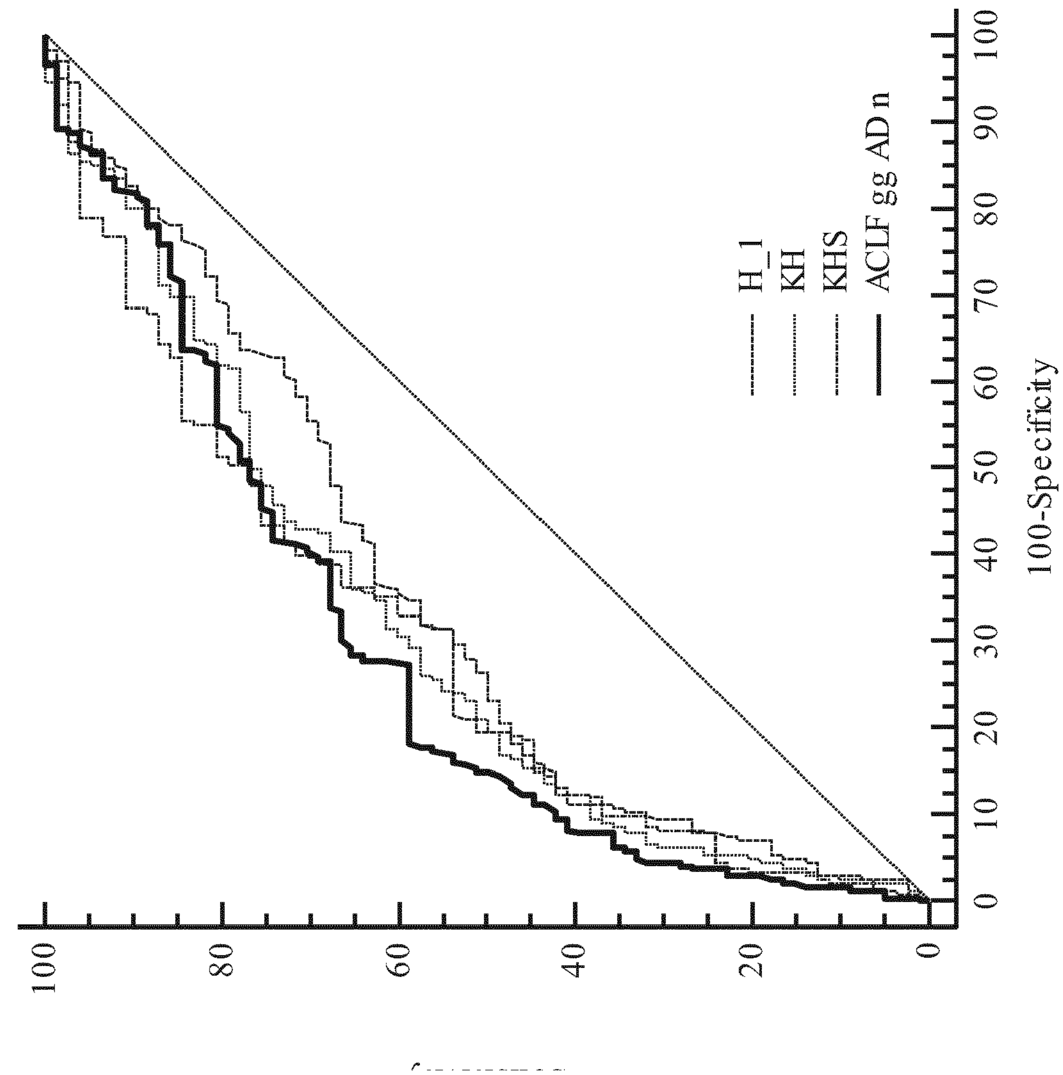
H_1
KH
KHS
ACLF gg AD n
100-Specificity
0
10
20
30
40
50
60
70
80
90
100
0
20
40
60
80
100

Fig. 13

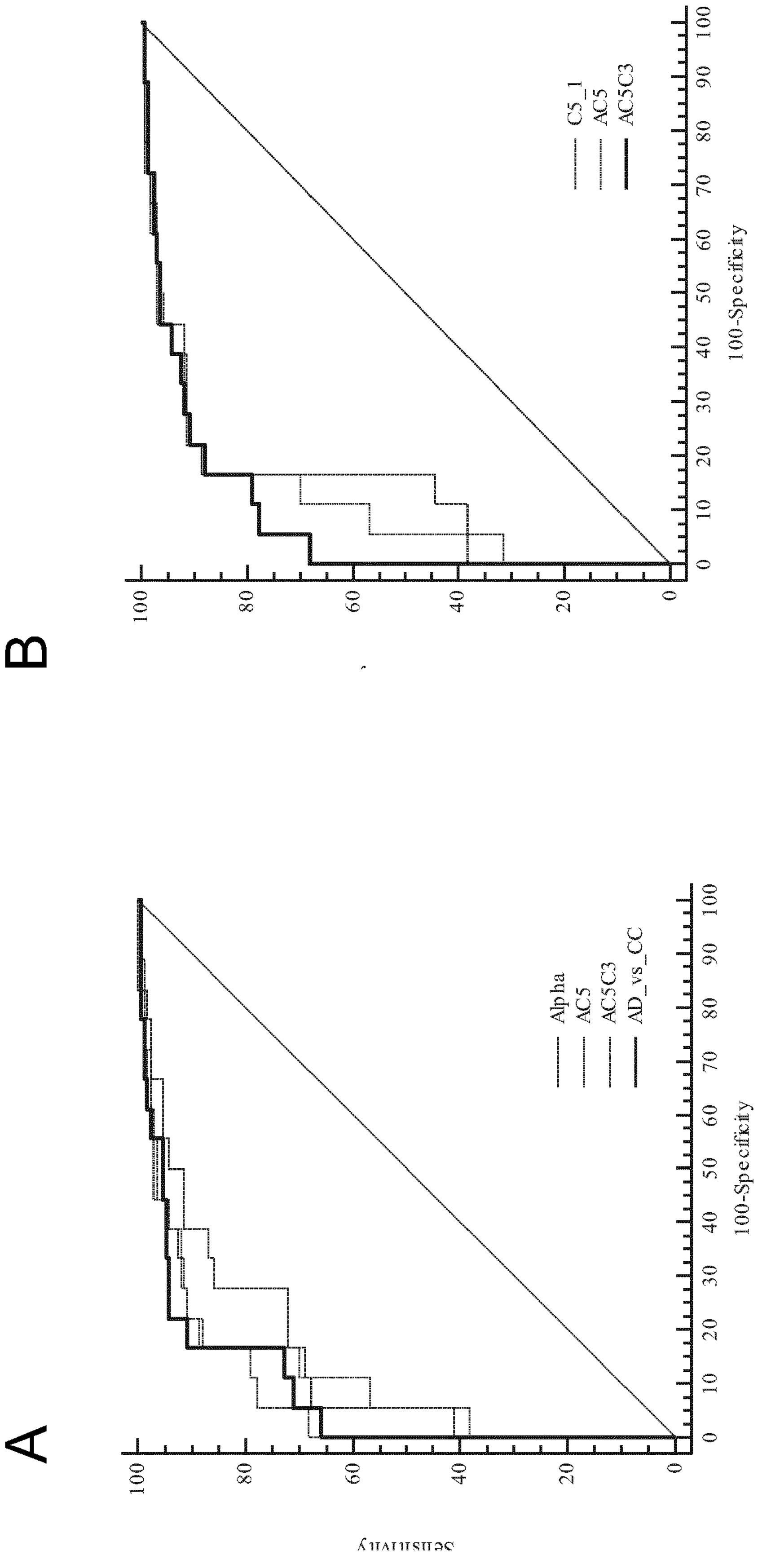
A
B
Alpha
AC5
AC5C3
AD_vs_CC
C5_1
AC5
AC5C3
100-Specificity
100-Specificity
0
20
40
60
80
100
0
10
20
30
40
50
60
70
80
90
100

Fig. 14

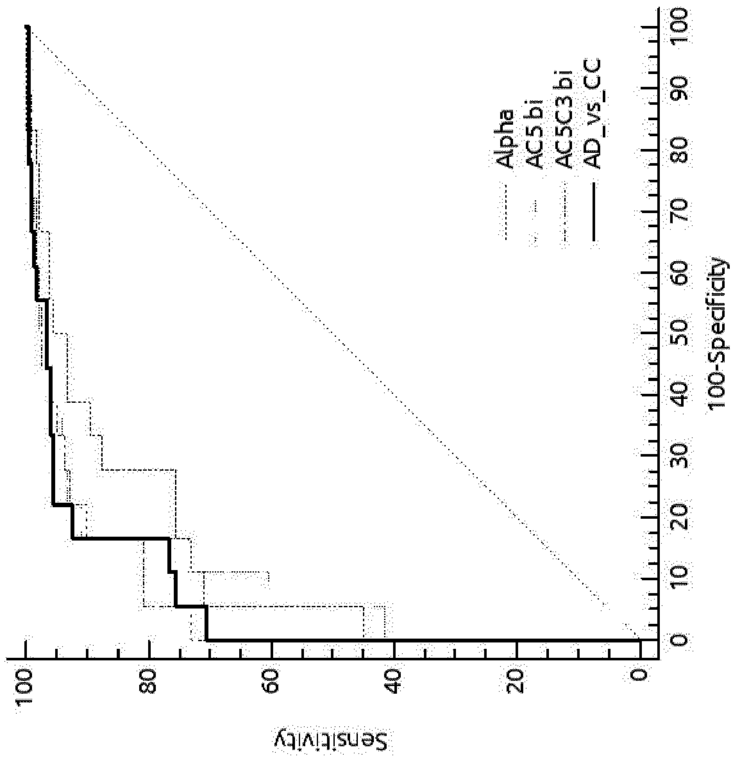
Alpha
AC5 bi
AC5C3 bi
AD_vs_CC
Sensitivity
100-Specificity
0
20
40
60
80
100
0
10
20
30
40
50
60
70
80
90
100

Fig. 15

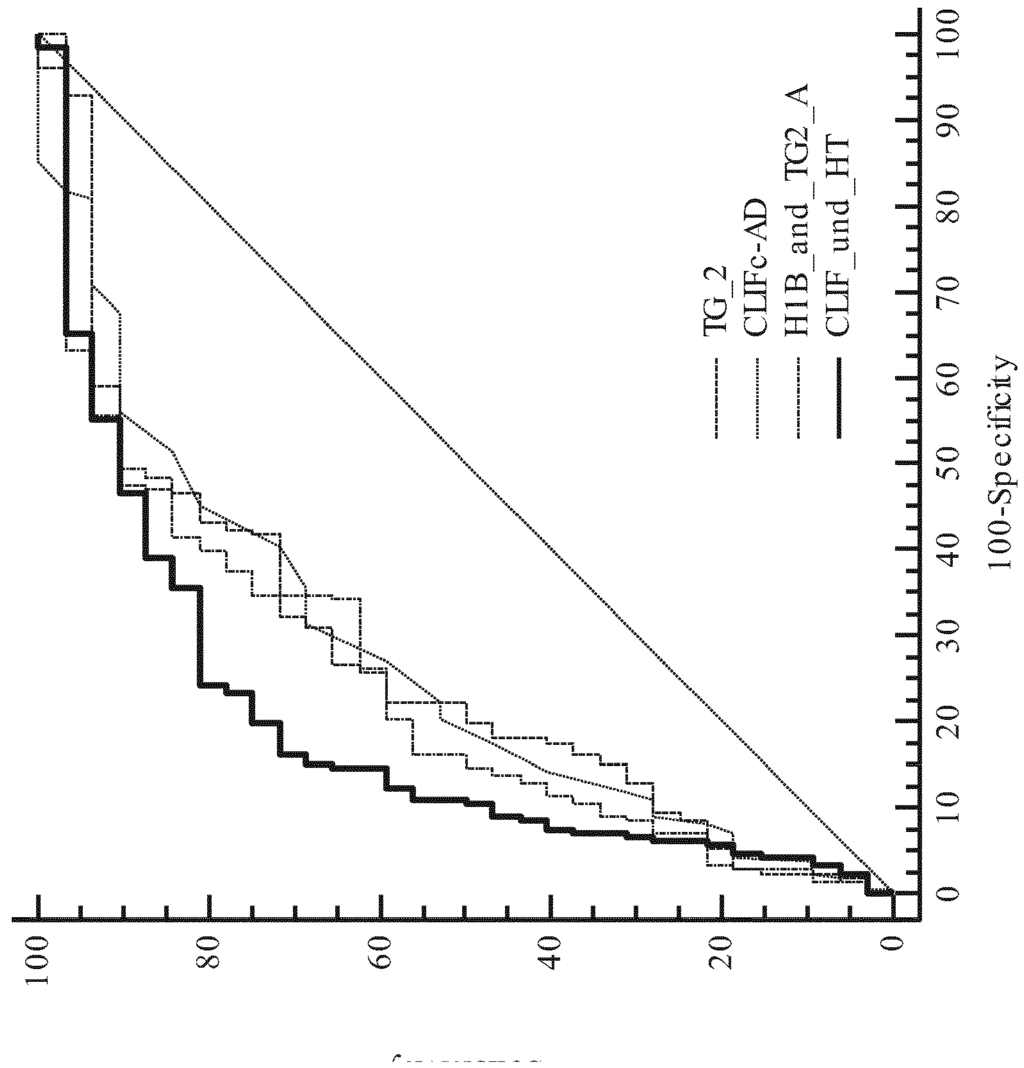
TG_2
CLIFc-AD
H1B_and_TG2_A
CLIF_und_HT
100-Specificity
0
10
20
30
40
50
60
70
80
90
100
0
20
40
60
80
100

Fig. 16

Europäisches Patentamt
European Patent Office
Office européen des brevets

# EUROPEAN SEARCH REPORT

Application Number
EP 19 19 4752

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/050148 A2 (UCL BUSINESS PLC [GB]; DAVIES NATHAN [GB]; JALAN RAJIV [GB]) 2 May 2008 (2008-05-02) * Example 1 * ----- | 1-15 | INV. G01N33/68 G01R33/60 |
| Y | RAJIV JALAN ET AL: "Alterations in the functional capacity of albumin in patients with decompensated cirrhosis is associated with increased mortality", HEPATOLOGY, vol. 50, no. 2, 18 February 2009 (2009-02-18), pages 555-564, XP55664957, ISSN: 0270-9139, DOI: 10.1002/hep.22913 * abstract; p.557; table 2 * ----- | 1-15 | |
| Y | STEVEN C KAZMIERCZAK ET AL: "Electron Spin Resonance Spectroscopy of Serum Albumin: A Novel New Test for Cancer Diagnosis and Monitoring", CLINICAL CHEMISTRY, vol. 52, no. 11, 1 January 2006 (2006-01-01), pages 2129-2134, XP055512356, DOI: 10.1373/dinchem.2006.073148 * abstract; p.557; table 2 * ----- | 1-15 | TECHNICAL FIELDS SEARCHED (IPC) G01N G01R |

The present search report has been drawn up for all claims

| Place of search | Date of completion of the search | Examiner |
|---|---|---|
| Munich | 4 February 2020 | Hohwy, Morten |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 19 4752

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2020

| Patent document cited in search report | Publication date | Patent family member(s) | Publication date |
|---|---|---|---|
| WO 2008050148 A2 | 02-05-2008 | CN 101541357 A | 23-09-2009 |
| | | EP 2061533 A2 | 27-05-2009 |
| | | JP 5276002 B2 | 28-08-2013 |
| | | JP 2010507432 A | 11-03-2010 |
| | | US 2010025328 A1 | 04-02-2010 |
| | | WO 2008050148 A2 | 02-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1269213 B1
- WO 0165270 A
- US 2003170912 A1
- US 7166474 B
- WO 2000004387 A3


### Non-patent literature cited in the description


- **JALAN, R. et al.** Alterations in the Functional Capacity of Albumin in Patients with Decompensated Cirrhosis is Associated with Increased Mortality. *Hepatology,* 2009, vol. 50, 555-564
- **MURAVSKY, V. ; GURACHEVSKAYA, T. ; BEREZENKO, S. ; SCHNURR, K. ; GURACHEVSKY, A.** Fatty acid binding sites of human and bovine albumins: differences observed by spin probe ESR. *Spectrochim Acta A Mol Biomol Spectrosc,* 2009, vol. 74, 42-47
- **BHATTACHARYA et al.** *J Mol Biol.,* 10 November 2000, vol. 303 (5), 721-32
- **SIMARD et al.** *J Mol Biol.,* 11 August 2006, vol. 361 (2), 336-51
- **CURRY et al.** *Nat Struct Biol.,* September 1998, vol. 5 (9), 827-35
- **GURACHEVSKY et al.** *Biochem Biophys Res Commun.,* 07 September 2007, vol. 360 (4), 852-6
- **ANDREY GURACHEVSKY ; EKATERINA SHIMANOVITCH ; TATJANA GURACHEVSKAYA ; VLADIMIR MURAVSKY.** Intra-albumin migration of bound fatty acid probed by spin label ESR. *Biochemical and Biophysical Research Communications,* 2007, vol. 360, 852-856